# EUROPÄISCHE PATENTSCHRIFT

(11) **EP 2 951 162 B1**
(45) Veröffentlichungstag und Bekanntmachung des Hinweises auf die Patenterteilung: **09.11.2016**
(21) Anmeldenummer: 14701397.3
(22) Anmeldetag: 27.01.2014
(51) Int. Cl.: C07D 251/24, C08G 18/81

(54) **UV-ABSORBER ENTHALTENDES URETHANACRYLAT**
UV ABSORBER CONTAINING URETHANE ACRYLATE
ABSORBEUR À UV CONTENANT DE L'ACRYLATE D'URÉTHANE

(30) Priorität: 01.02.2013 EP 13153700
(43) Veröffentlichungstag der Anmeldung: 09.12.2015
(73) Patentinhaber: Covestro Deutschland AG, 51373 Leverkusen (DE)
(72) Erfinder: KOSTROMINE, Serguei, 53913 Swisttal-Buschhoven (DE); PETZOLDT, Joachim, 40789 Monheim (DE); FISCHER, Wolfgang, 40668 Meerbusch (DE)
(74) Vertreter: Levpat
(86) Internationale Anmeldenummer: PCT/EP2014/051489
(87) Internationale Veröffentlichungsnummer: WO 2014/118116

(56) Entgegenhaltungen:
- WO-A1-2011/006552
- WO-A1-2012/049091

## Beschreibung

Die vorliegende Erfindung betrifft ein UV-Absorber enthaltendes Urethanacrylat, ein Verfahren zu seiner Herstellung und dessen Anwendung. Der UV-Absorber ist hierbei chemisch in das System angebunden.

Für Außenanwendungen müssen transparente Kunststoffartikel, wie z.B. Platten, Folien oder Extrusionsformkörper, vor allem mittels UV-Schutz vor der aggressiven Sonnenstrahlung und mittels Kratzfestausrüstung vor mechanischen Einwirkungen geschützt werden. Ein gängiges Verfahren dafür ist die obere und manchmal auch die einzige Schutzschicht, die kratzfest sein muss, zusätzlich mit UV-Schutzfunktion zu versehen und dafür mit einer wesentlichen Menge von UV-Absorbern zu bestücken (vgl. DE-A 10 2006 016 642). Die klassischen UV-Absorber wirken allerdings in den Schutzschichten als Weichmacher und verringern die mechanische Beständigkeit der Schicht.

Typische UV-Absorber-Klassen sind beispielsweise Biphenyl-substituierte Triazine (vgl. WO-A 2006/108520). Diese Substanzklasse zeigt eine hervorragende Absorptionswirkung bei 320 - 380 nm und gleichzeitig eine sehr hohe eigene UV-Stabilität (WO 2000/066675 A1, US-A 6,225,384).

Der Einfluss des UV-Absorbers auf die mechanische und chemische Beständigkeit der Beschichtung ist umso größer, desto höher seine Konzentration in der Beschichtung ist. Man benötigt aber gewisse Konzentrationen des Absorbers, um UV-Licht effektiv zu absorbieren und der Substrat davon sicher und dauerhaft zu schützen. Diese Konzentration wird laut Lambert-Gesetz je höher sein, desto dünner die Beschichtung ist. Für die modernen Folienbeschichtungen, deren Schichtstärke zwischen 1 und 10 µm liegen können, bedeutet es, dass sie bis zu 10 Gew.-% des UV-Absorbers beinhalten sollten, um die notwendige Absorptionskraft aufzuweisen. Die mechanische und chemische Beständigkeit solcher Beschichtungen könnte aber dadurch negativ beeinträchtigt werden

Eine Lösung des Problems könnte es sein, wenn der UV-Absorber nicht als passives Additiv vorliegt, sondern in dem Härtungsprozess chemisch aktiv teilnimmt und sich ins Polymergerüst der Beschichtung einbindet.

Die vorliegende Erfindung hat sich die Aufgabe gestellt, solche UV-Absorber und ein Verfahren zu ihrer Herstellung bereitzustellen. Zusammenfassend wurde diese Aufgabe dadurch gelöst, dass man UV-Absorber aus der Klasse der Biphenyl-substituierten s-Triazine an die Struktur von Urethanacrylat-Harzen chemisch angebunden hat.

Erfindungsgemäß gelöst wurde die Aufgabe durch ein UV-Absorber enthaltendes Urethanacrylat der Formel (I): 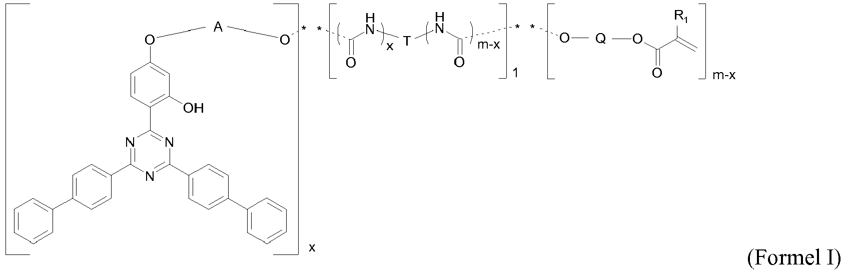 wobei
***R₁*** ein Wasserstoff oder ein Methylrest ist,
***Q*** ein Linker des handelsüblichen Hydroxyalkyl(Meth)acrylates aus der Gruppe von 2-Hydroxyethyl(meth)acrylat, 2-Hydroxypropyl(meth)acrylat, 4-Hydroxybutyl(meth)acrylat, 3-Hydroxy-2,2-dimethylpropyl(meth)acrylat ist,
T ein Kern der handelsüblichen aliphatischen und cycloaliphatischen Polyisocyanate T(NCO)*ₘ* ist, die cyclische Isocyanurat-, Uretdion-, Iminooxadiazindion- oder Oxadiazintrion-Strukturen aufweisen, ebenso wie verzweigte Biuret-Strukturen in Falle der cycloaliphatischen Polyisocyanate aufweisen.
***m*** dabei die ursprüngliche durchschnittliche NCO-Funktionalität des angewendeten Polyisocyanates entspricht und gleich oder mehr als 2 ist,
***A*** für einen gegebenenfalls substituierten linearen oder verzweigten Linker aus Kohlenstoff, Sauerstoff, Stickstoff, Schwefel, Phosphor und/oder Silizium in der Kette steht
   und
***x*** für ein durchschnittlicher molaren Anteil des angebundenen UV-Absorber-Restes steht und weniger als ***m*** ist. Bevorzugt ist x gleich oder weniger als 1.

Im Sinne des durchschnittlichen Wertes von ***m*** und **x** sind auch die Mischungen der eine bis mehreren Strukturen der Formel (I) mit Strukturen der Formeln (II) und (III), 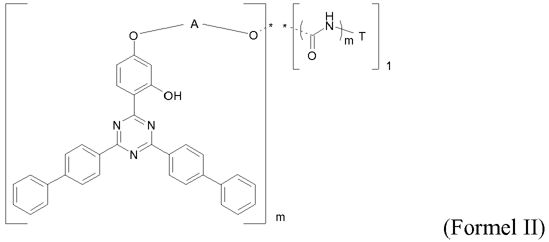 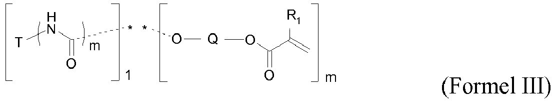 deren Erscheinung in Herstellungsprozess der Produkte von Formel (I) nicht ausgeschlossen sein kann, auch erfindungsgemäß.

Die erfindungsgemäßen Verbindungen der Formel (I) weisen bevorzugt ein UV-Absorptionsmaximum zwischen 300 und 340 nm auf.

Bevorzugt steht A in den Verbindungen der allgemeinen Formel (I) für einen gegebenenfalls substituierten linearen oder verzweigten Linker, wobei zwischen dem O-Atom der aromatische Kern des UV-Absorbers und dem O-Atom der Urethan-Gruppe eine Kette aus mindestens 4 Atomen ausgewählt aus Kohlenstoff, Sauerstoff, Stickstoff, Schwefel, Phosphor und/oder Silizium in der Kette besteht.

Ausführungsformen und weitere Aspekte der vorliegenden Erfindung werden nachfolgend beschrieben. Sie können beliebig miteinander kombiniert werden, sofern sich aus dem Kontext nicht eindeutig das Gegenteil ergibt.

In einer Ausführungsform des erfindungsgemäßen UV-Absorber enthaltenden Urethanacrylats ist das Urethanacrylat gemäß Formel (I-1) aufgebaut: 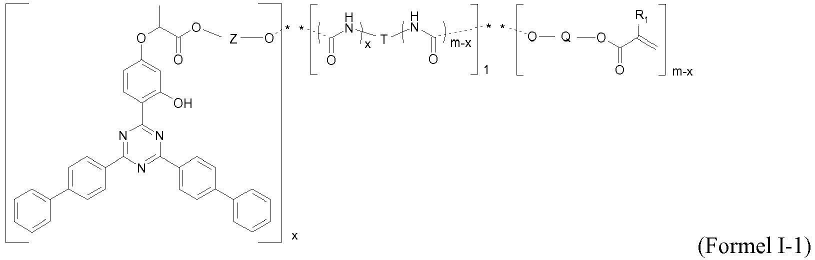 worin
Z für einen gegebenenfalls substituierten linearen oder verzweigten C₁₋₂₀-Alkylen-Rest oder C₁₋₂₀-Alkylenether-Rest steht, und
T, Q, m, x und R₁ die vorangehend für die Verbindungen der allgemeinen Formel (I) genannte Bedeutung haben.

Die ganz besonders bevorzugten Harze Formel (I-1) sind diejenige, die aus Substanzen der Formel (IV-4; s. unten) hergestellt sind:

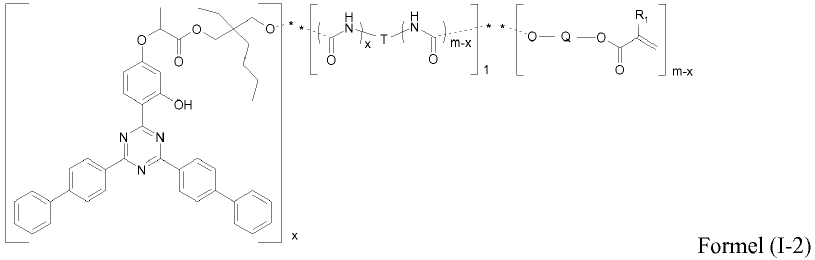

Ein weiterer Gegenstand der vorliegenden Erfindung ist ein Verfahren zur Herstellung eines UV-Absorber enthaltenden Urethanacrylats, umfassend die Schritte:
a) Reaktion einer Verbindung der allgemeinen Formel: 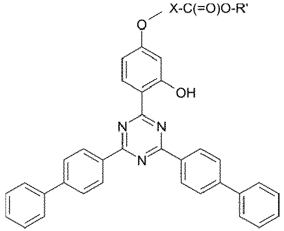 wobei X für verzweigtes oder unverzweigtes C₁₋₂₀-Alkyl steht und R' für verzweigtes oder unverzweigtes C₁₋₂₀-Alkyl, C₄₋₁₂-Cycloalkyl oder gegebenenfalls durch C₁₋₁₂-Alkyl, C₁₋₁₂-Alkoxy, CN und/oder Halogen substituiertes C₆₋₁₂-Aryl steht
   mit einem mindestens difunktionellen Alkohol;
b) Reaktion des in Schritt a) erhaltenen Produkts mit
   bi) einem aliphatischen oder cycloaliphatischen, Isocyanatgruppen enthaltenden Urethanacrylat, welches cyclische Isocyanurat-, Uretdion-, Iminooxadiazindion- oder Oxadiazintrion-Strukturen aufweist oder, im Fall eines cycloaliphatischen Urethanacrylats, weiterhin verzweigte Biuret-Strukturen aufweisen kann,
      und/oder mit
   bii) einem aliphatischen oder cycloaliphatischen, Isocyanatgruppen enthaltenden Polyisocyanat, welches cyclische Isocyanurat-, Uretdion-, Iminooxadiazindion- oder Oxadiazintrion-Strukturen aufweist oder, im Fall eines cycloaliphatischen Polyisocyanat, weiterhin verzweigte Biuret-Strukturen aufweisen kann,
wobei die Reaktion in Schritt b) weiterhin in Gegenwart eines Hydroxyalkyl(meth)acrylats erfolgt und/oder nach der Reaktion in Schritt b) das erhaltene Produkt weiter mit einem Hydroxyalkyl(meth)acrylat umgesetzt wird.

Im erfindungsgemäßen Verfahren kann beispielsweise ein Polyisocyanat T(NCO)*ₘ* zur Reaktion mit der in einem passenden Lösungsmittel gelösten Substanz Formel (IV) gebracht werden: 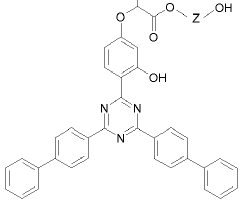 (Formel IV)wobei Z für lineares oder verzweigtes Alkyl C₁₋₂₀ oder C₁₋₂₀ Alkylenether steht.

Diese Reaktion wird so lange geführt, bis die ganze Substanz Formel (IV) durch entstehende Urethan-Gruppen mit Polyisocyanat gebunden ist. Dann wird dem Harz Hydroxyalkyl(meth)acrylat zugegeben, um die alle restlichen NCO-Gruppen von Polyisocyanat mit OH-Gruppen des Hydroxyalkyl(meth)acrylats abreagieren zu lassen. Am Ende dieser Reaktion wird das passende Lösemittel zugegeben, um die Viskosität des Harzes auf ein gewünschtes Niveau zu bringen.

Ein besonderer Vorteil des Verfahrens besteht darin, dass die Substanz der Formel (IV) eine primäre OH-Gruppe besitzt und in die Reaktion mit Isocyanat-Gruppen ohne Katalysator, besonders ohne Zinn-Katalysator und ganz besonders ohne Dibutylzinndilaurat(DBTL)-Katalysator eingehen kann. Durch das erfindungsgemäße Verfahren entstehen also Zinn-freie, besonders vorteilhaft DBTL-freie Produkte der Formel (I).

Dieses unterscheidet das erfindungsgemäße Verfahren von dem Verfahren aus DE 10 2009 032 921 A1 (BASF), wo die Triazin-Absorber mit sekundären OH-Gruppen angewendet wurden. Dieses erfordert die Anwendung von sehr großen Mengen von DBTL in Prozent-Bereich. Außerdem betrifft es nur Phenyl-Triazine und keine besonders vorteilhafte Biphenyl-substituierte Triazine. Ein weiterer wichtiger Unterschied des erfindungsgemäßen Verfahrens liegt in Verwendung der Alkohole als Lösemittel für Produkte Formel (I), was sie besonders vorteilhaft für Beschichtung von Polycarbonat macht. Das Verfahren aus DE 10 2009 032 921 benutzt MEK, welches Polycarbonat sofort angreift.

Die mit einer OH-Gruppe funktionalisierten UV-Absorber s-Triazin-Klasse (Formel IV) werden gemäß US 2012/0094127 A1 durch eine direkte Umesterung von Tinuvin 479® (Produkt Fa. BASF) mit einem Diol HO-Z-OH hergestellt.

Eine weitere erfindungsgemäße Ausführung des Herstellungsverfahren besteht darin, dass anstatt der Polyisocyanate T(NCO)ₘ die NCO-haltigen Urethanacrylate (Formel V): 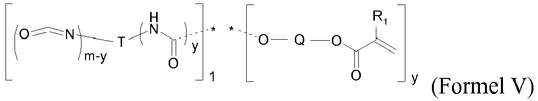 eingesetzt werden. Beispiele solcher Produkte sind die NCO-haltigen Urethanacrylate der Fa. Bayer MaterialScience: Desmolux® D100, VP LS 2396 und XP 2510. Der weitere Verlauf des Verfahrens bleibt dabei wie oben beschrieben.

Die Substanz der Formel IV wird in einer unter den Reaktionsbedingungen flüssigen Form in die Reaktion mit Polyisocyanat eingeführt. Drei Varianten stehen dabei zur Betrachtung:
- Die Substanz der Formel IV wird in Form einer Lösung in einem weiteren Reagenz des Verfahrens wie Hydroxyalkyl(meth)acrylat dem Polyisocyanat zugegeben. Die restliche Menge von Hydroxyalkyl(meth)acrylat kommt zur Reaktion später als zweiter Schritt;
- Die Substanz der Formel IV wird in Form einer Lösung in NCO-neutralem Lösemittel dem Polyisocyanat zugegeben. Das Hydroxyalkyl(meth)acrylat kommt dann in der ganzen Menge zur Reaktion später als zweiter Schritt. Die Tatsache, dass die Reaktion NCO-Gruppe mit OH-Gruppe erfindungsgemäß ohne Katalysator abläuft, erlaubt die Verwendung von tertiären Alkohole, wie z.B. Diacetonalkohol, als Lösemittel (besonders vorteilhaft) für diese Synthese.
- Die Substanz der Formel IV reagiert in Form einer Schmelze mit dem Polyisocyanat. Das Hydroxyalkyl(meth)acrylat kommt dann später als zweiter Schritt in der ganzen Menge zur Reaktion.

Die erste Variante des Verfahrens eignet sich gut zur Herstellung des Harzes Formel (I) und (I-1) mit relativ kleinem x-Wert (x<0,1), wobei nur relativ kleine Menge der Substanz Formel IV gelöst und zur Reaktion gebracht werden soll.

Weitere Variationen des Verfahrens sind mit der Auswahl der benutzten Lösemittel und mit der Reihenfolge deren Anwendung verbunden. Die einzige Einschränkung dabei ist, dass genutztes Lösemittel (hier bezeichnet als Lösemittel der Gruppe 1) unter den Verfahrensbedingungen mit Isocyanat-Gruppen nicht reagiert. Typische Beispiele sind dem Fachmann bekannt. Dies sind aromatische Kohlenwasserstoffe (Toluol) cyclische Ether (THF, Dioxan), Ketone (Aceton, MEK, Cyclopentanon) oder Ester (Ethyl- und Butylacetat).

Die Lösemittel der Gruppe 2, die am Ende des Verfahrens ins Reaktionsgemisch kommen, wenn das Harz Formel (I) keine freie NCO-Gruppen mehr besitzt, unterlegen den Einschränkungen der Gruppe 1 nicht mehr. Zu der Gruppe 2 gehören also alle Lösemittel der Gruppe 1 plus alle dem Fachmann bekannten OH-haltigen Lösemittel, wie z.B. Ethanol, Isopropanol, Butanol, 1-Methoxy-2-propanol, sowie deren Mischungen.

In einer Ausführungsform des erfindungsgemäßen Verfahrens steht in Schritt a) in der allgemeinen Formel X für CH(CH₃).

In einer weiteren Ausführungsform des erfindungsgemäßen Verfahrens steht in Schritt a) in der allgemeinen Formel R' für n-Octyl oder iso-Octyl.

In einer weiteren Ausführungsform des erfindungsgemäßen Verfahrens ist in Schritt a) der mindestens difunktionelle Alkohol ausgewählt aus der Gruppe 2-Butyl-2-ethyl-1,3-propandiol, 2,2-Diethyl-1,3-propandiol, 2-Methyl-1,3-propandiol und/oder 2,2-Dimethyl-1,3-propandiol.

In einer weiteren Ausführungsform des erfindungsgemäßen Verfahrens ist das aus Schritt a) erhaltene Produkt ausgewählt aus:

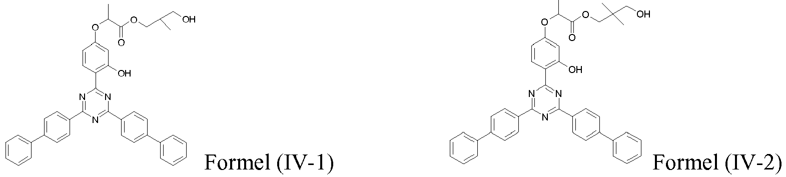

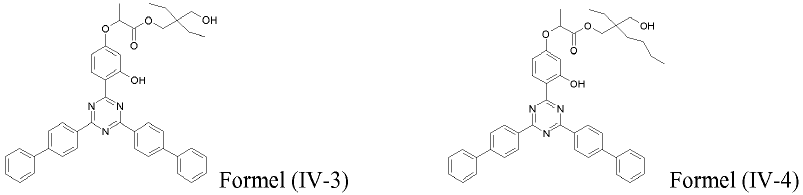

Die Substanzen der Formeln (IV-1) bis (IV-3) sind kristalline Substanzen mit Schmelzpunkten, die über 100 °C liegen. Das macht die Verwendung der Lösemittel der Gruppe 1 bei der Herstellung der Harze Formel (I-1) sinnvoll. Die Tatsache, dass die Reaktion der NCO-Gruppen mit den OH-Gruppen erfindungsgemäß ohne Katalysator abläuft, erlaubt die Verwendung tertiärer Alkohole, wie z.B. Diacetonalkohol, als Lösemittel (besonders vorteilhaft) für diese Synthese.

Die Substanz der Formel (IV-4) hat einen Schmelzpunkt von ca. 80 °C. Diese Substanz kann als Schmelze in Herstellungsprozess der Harze Formel (I-1) vorliegen. Lösemittel der Gruppe 1 werden bei der Herstellung nicht gebraucht, was als vorteilhaft bewertet werden kann.

In einer weiteren Ausführungsform des erfindungsgemäßen Verfahrens ist in Schritt b) das Isocyanatgruppen enthaltende Urethanacrylat erhältlich durch die Reaktion eines 1,6-Hexamethylendiisocyanat-Isocyanurats mit einem Hydroxyalkyl(meth)acrylat.

In einer weiteren Ausführungsform des erfindungsgemäßen Verfahrens ist in und/oder nach Schritt b) das Hydroxyalkyl(meth)acrylat ausgewählt aus der Gruppe 2-Hydroxyethyl(meth)acrylat, 2-Hydroxypropyl(meth)acrylat, 4-Hydroxybutyl(meth)acrylat und/oder 3-Hydroxy-2,2-dimethylpropyl(meth)acrylat.

Die vorliegende Erfindung betrifft weiterhin eine Beschichtungszusammensetzung, umfassend ein UV-Absorber enthaltendes Urethanacrylat gemäß der Erfindung und/oder ein durch ein Verfahren gemäß der Erfindung erhältliches, UV-Absorber enthaltendes Urethanacrylat.

In einer Ausführungsform der erfindungsgemäßen Beschichtungszusammensetzung umfasst diese:
i) UV-Absorber enthaltendes Urethanacrylat gemäß der vorliegenden Erfindung und/oder ein durch ein Verfahren gemäß der vorliegenden Erfindung erhältliches, UV-Absorber enthaltendes Urethanacrylat in 0,1 bis 50 Gew.-Teilen (vorzugsweise 4 bis 8 Gewichts-%, bezogen auf die Gesamtmenge der Beschichtungszusammensetzung);
ii) 12 bis 70 Gew.-Teile wenigstens eines C₂-C₁₂-Diol-Diacrylats oder C₂-C₁₂-Diol-Dimethacrylats, worin C₂-C1₂ für einen linearen Alkylenrest steht, der gegebenenfalls mit einer Methylgruppe substituiert sein kann oder durch ein oder mehrere Sauerstoff-Atom(e) unterbrochen und optional mit einer oder mehreren Methylgruppe(n) substituiert sein kann,
iii) 12 bis 40 Gew.-Teile wenigstens eines alkoxylierten, bevorzugt ethoxylierten Mono-, Di-, Tri-, Tetra-, Penta- oder Hexaacrylats oder alkoxylierten, bevorzugt ethoxylierten Mono-, Di-, Tri-, Tetra-, Penta- oder Hexamethacrylats,
iv) 0 bis 40 Gew.-Teile wenigstens eines Monomeren ausgewählt aus der Gruppe umfassend Pentaerythrit-Triacrylat, Pentaerythrit-Tetraacrylat, Dipentaerythrit-Tetraacrylat, Dipentaerythrit-Pentaacrylat Dipentaerythrit-Hexaacrylat, Pentaerythrit-Trimethacrylat, Pentaerythrit-Tetramethacrylat, Dipentaerythrit-Tetramethacrylat, Dipentaerythrit-Pentamethacrylat, Dipentaerythrit-Hexamethacrylat und deren möglichen Reaktionsprodukten mit aliphatischen oder aromatischen Diisocyanaten,
v) 5 bis 60 Gew.-Teile wenigstens eines weiteren Mono-, Di- oder Triacrylats oder Mono-, Di- oder Trimethacrylats,
   wobei sich die Gewichtsteile der Komponenten i) bis v) zu ≤ 100 Gew.-Teilen addieren, und die Lackzusammensetzung zusätzlich wenigstens noch
vi) 0,1 bis 10 Gew.-Teile wenigstens eines Photoinitiators enthält.

Bei der Komponente ii) handelt es sich bevorzugt um wenigstens ein C4-C12-Diol-Diacrylat oder C4-C12-Diol-Dimethacrylat, besonders bevorzugt um wenigstens ein C4-C8-Diol-Diacrylat oder C4-C8-Diol-Dimethacrylat. Bei den C2-C12-, bevorzugt C4-C12-, besonders bevorzugt C4-C8-Einheiten handelt es sich bevorzugt um lineare Alkylenreste, welche gegebenenfalls mit einer Methylgruppe substituiert sein können oder durch ein oder mehrere Sauerstoff-Atom(e) unterbrochen und optional mit einer oder mehreren Methylgruppe(n) substituiert sein können. Bevorzugt handelt es sich um lineare Alkenreste, welche gegebenenfalls durch ein oder mehrere Sauerstoff-Atom(e) unterbrochen sein können.

Als geeignete Diol-Diacrylate oder Diol-Dimethacrylate kommen ganz besonders bevorzugt solche der allgemeinen Formel (VI) in Frage,

H2C=C(R2)-C(O)-O-(CH₂)ₙ-O-C(O)-C(R2)=CH₂ (Formel VI)

worin
R2 für H oder CH₃, bevorzugt für H steht, und
n für eine ganze Zahl von 2 bis 12, bevorzugt von 4 bis 12, besonders bevorzugt von 4 bis 8 steht.

Als geeignete Diol-Diacrylate oder Diol-Dimethacrylate kommen weiterhin ganz besonders bevorzugt solche der allgemeinen Formel (VII) in Frage,

H₂C=C(R3)-C(O)-O-(CHR4-CH₂-O)ₖ-C(O)-C(R3)=CH₂ (Formel VII)

worin
R3 für H oder CH3, bevorzugt für H steht,
R4 für H oder CH3, steht, und k für eine ganze Zahl von 2 bis 5, bevorzugt von 2 bis 4 steht.

Geeignete C4-C8-Diol-Diacrylate oder -Diol-Dimethacrylate sind beispielsweise Diethylenglycoldiacrylat, Diethylenglycoldimethacrylat, Triethylenglycoldiacrylat, Triethylenglycoldimethacrylat, Tetraethylenglykoldiacrylat, Tetraethylenglykoldimethacrylat, Dipropylenglykoldiacrylat, Dipropylenglykoldimethacrylat, Tripropylenglykoldiacrylat, Tripropylenglykoldimethacrylat, 1,4-Butandioldiacrylat, 1,4-Butandioldimethacrylat, 1,5-Pentandioldiacrylat, 1,5-Pentandioldimethacrylat, 1,6-Hexandioldiacrylat, 1,6-Hexandioldimethacrylat, 3-Methyl-1,5-Pentandioldiacrylat, 3-Methyl-1,5-Pentandioldimethacrylat, 1,7-Heptandioldiacrylat, 1,7-Heptandioldimethacrylat, 1,8-Octandioldiacrylat und/oder 1,8-Octandioldimethacrylat. Darüber hinaus geeignete C4-C12-Diol-Diacrylate oder -Diol-Dimethacrylate sind beispielsweise 1,9-Nonandioldiacrylat, 1,9-Nonandioldimethacrylat, 2-Methyl-1,8-Octandioldiacrylat, 2-Methyl-1,8-Octandioldimethacrylat, 1,10-Decandioldiacrylat, 1,10-Decandioldimethacrylat, 1,11-Undecandioldiacrylat, 1,11-Undecandioldimethacrylat, 1,12-Dodecandioldiacrylat und/oder 1,12-Dodecandioldimethacrylat. Darüber hinaus geeignete C2-C12-Diol-Diacrylate oder -Diol-Dimethacrylate sind beispielsweise Ethylenglycoldiacrylat, Ethylenglycoldimethacrylat, 1,3-Propandioldiacrylate und/oder 1,3-Propandioldimethacrylat. Ganz besonders bevorzugt sind die jeweiligen Diacrylate. Besonders bevorzugt sind 1,6-Hexandioldiacrylat und/oder 1,6-Hexandioldimethacrylat Diethylenglycoldiacrylat, Diethylenglycoldimethacrylat, insbesondere bevorzugt 1,6-Hexandioldiacrylat Diethylenglycoldiacrylat.

Die Lackzusammensetzung enthält bevorzugt 12 bis 35 Gew.-Teile, besonders bevorzugt 15 bis 30 Gew.-Teile, ganz besonders bevorzugt 20 bis 30 Gew.-Teile der Komponente iii). Bei den genannten Gewichtsteilen handelt es sich um die Summe der Gewichtsteile aller alkoxylierten Mono-, Di-, oder Triacrylate oder -methacrylate, aus denen sich die Komponente iii) zusammensetzt.

Bei den alkoxylierten Monoacrylaten oder -methacrylaten für die Komponente (iii) kann es sich um alkoxylierte gegebenenfalls substituierte aliphatische, cycloaliphatische, aromatische, gemischt aromatisch-aliphatische Monoacrylate oder -methacrylate handeln. Dabei kommen sowohl alkoxylierte lineare als auch verzeigte aliphatische Monoacrylate oder -methacrylate in Frage, in denen die Alkylkette zudem durch ein oder mehrere Heteroatome, wie z.B. Sauerstoffatome unterbrochen sein kann. Im Falle der cycloaliphatischen oder aromatischen Monoacrylate oder - methacrylate kommen auch heterocyclische oder heteroaromatische Monoacrylate oder - methacrylate in Frage.

Beispiele für solche alkoxylierten Monoacrylate oder -methacrylate sind alkoxyliertes, bevorzugt ethoxyliertes Methylacrylat, Ethylacrylat, n-Butylacrylat, Isobutylacrylate, t-Butylacrylat, 2-Ethyl-hexylacrylat, Isodecylacrylat, n-Laurylacrylat, C12-C15-Alkylacrylate, n-Stearylacrylat, n-Butoxyethylacrylat, Butoxydiethylenglycolacrylat, Methoxytriethylenglycolacrylat, Cyclohexylacrylat, Tetrahydrofurfurylacrylat, Benzylacrylat, 2-Phenoxyethylacrylat, Isobornylacrylat, 2-Hydroxyethylacrylat, 2-Hydroxypropylacrylat, 2-Hydroxyethylacrylat, 2-Hydroxybutylacrylat, 2-Hydroxybutylacrylat und die entsprechenden alkoxylierten, bevorzugt ethoxylierten Methacrylate.

Bei den alkoxylierten Diacrylaten oder -methacrylaten für die Komponente (iii) kann es sich beispielsweise um solche handeln, die von den Diol-Diacrylaten und -methacrylaten der Komponente ii) verschieden sind.

Beispiele für solche alkoxylierten Diacrylate oder -methacrylate sind alkoxyliertes, bevorzugt ethoxyliertes Methandioldiacrylat, Methandioldimethacrylat, Glycerindiacrylat, Glycerindimethacrylat, Neopentylglycoldiacrylat, Neopentylglycoldimethacrylat, 2-Butyl-2-Ethyl-1,3-Propandioldiacrylate, 2-Butyl-2-Ethyl-1,3-Propandioldimethacrylat, Trimethylolpropandiacrylat oder Trimethylolpropandimethacrylat.

Beispiele für alkoxylierte Triacrylate oder -methacrylate für die Komponente (iii) sind alkoxyliertes, bevorzugt ethoxyliertes Pentaerythrit-Triacrylat, Pentaerythrit-Trimethacrylat, Glycerintriacrylat, Glycerintrimethacrylat, 1,2,4-Butantrioltriacrylat, 1,2,4-Butantrioltrimethacrylat, Trimethylolpropantriacrylat, Trimethylolpropantrimethacrylat, Tricyclodecandimethanoldiacrylat, Tricyclodecandimethanoldimethacrylat, Ditrimethylolpropantetraacrylat oder Ditrimethylolpropantetramethacrylat.

Beispiele für alkoxylierte Tetra-, Penta- oder Hexaacrylate sind alkoxyliertes, bevorzugt ethoxyliertes Pentaerythrit-Tetraacrylat, Dipentaerythrit-Tetraacrylat, Dipentaerythrit-Pentaacrylat Dipentaerythrit-Hexaacrylat, Pentaerythrit-Tetramethacrylat, Dipentaerythrit-Tetramethacrylat, Dipentaerythrit-Pentamethacrylat oder Dipentaerythrit-Hexamethacrylat.

In den alkoxylierten Diacrylaten oder -methacrylaten, Triacrylaten oder -methacrylaten, Tetraacrylaten oder -methacrylaten, Pentaacrylaten oder -methacrylaten und/oder alkoxylierten Hexaacrylaten oder -methacrylaten der Komponente iii) können alle Acrylatgruppen oder Methacrylatgruppen oder nur ein Teil der Acrylatgruppen oder Methacrylatgruppen im jeweiligen Monomer über Alkylenoxidgruppen an den entsprechenden Rest gebunden sein. Es können auch beliebige Mischungen solcher ganz oder teilweise alkoxylierten Di-, Tri-, Tetra-, Penta- oder Hexaacrylate bzw. -methacrylate eingesetzt werden. Dabei ist es auch möglich, das die Acrylat- oder Methacrylatgruppe(n) über mehrere aufeinanderfolgende Alkylenoxidgruppen, vorzugsweise Ethylenoxidgruppen an den aliphatischen, cycloaliphatischen oder aromatischen Rest des Monomeren gebunden sind. Die mittlere Anzahl der Alkylenoxid- bzw. Ethylenoxidgruppen im Monomer wird durch den Alkoxylierungsgrad bzw. Ethoxylierungsgrad angegeben. Der Alkoxylierungsgrad bzw. Ethoxylierungsgrad kann bevorzugt von 2 bis 25 betragen, besonders bevorzugt sind Alkoxylierungsgrade bzw. Ethoxylierungsgrade von 2 bis 15, ganz besonders bevorzugt von 3 bis 9.

Komponente (iii) enthält bevorzugt alkoxylierte, vorzugsweise ethoxylierte Di- und/oder Triacrylate. Komponente (iii) enthält besonders bevorzugt wenigstens ein alkoxyliertes, vorzugsweise ethoxyliertes Di- oder Triacrylat oder wenigstens ein alkoxyliertes, vorzugsweise ethoxyliertes Di- oder Trimethacrylat, ganz besonders bevorzugt ein ethoxyliertes Di- oder Triacrylat. Komponente (iii) enthält in bevorzugten Ausführungsformen der Erfindung wenigstens ein ethoxyliertes Triacrylat oder -methacrylat, vorzugsweise ethoxyliertes Triacrylat. Besonders bevorzugt enthält Komponente (iii) alkoxyliertes Trimethylolpropantriacrylat und/oder Trimethylolpropantrimethacrylat. In bevorzugten Ausführungsformen enthält Komponente (iii) ethoxyliertes Trimethylolpropantriacrylat und/oder Trimethylolpropantrimethacrylat, vorzugsweise ethoxyliertes Trimethylolpropantriacrylat. In bevorzugten Ausführungsformen beträgt der Ethoxylierungsgrad der Trimethylolpropantriacrylate und/oder Trimethylolpropantrimethacrylate 2 bis 25, besonders bevorzugt 2 bis 15, ganz besonders bevorzugt 3 bis 9.

Die Lackzusammensetzung enthält bevorzugt 0 bis 30 Gew.-Teile, besonders bevorzugt 0,1 bis 30 Gew.-Teile der Komponente iv). In besonders bevorzugten Ausführungsformen ist die Komponente III) in der Lackzusammensetzung enthalten. Bei den genannten Gewichtsteilen handelt es sich um die Summe der Gewichtsteile aller Monomere aus der genannten Gruppe, aus denen sich die Komponente iv) zusammensetzt.

Bei der Komponente iv) handelt es sich um Monomere ausgewählt aus der Gruppe umfassend Pentaerythrit-Triacrylat, Pentaerythrit-Tetraacrylat, Dipentaerythrit-Tetraacrylat, Dipentaerythrit-Pentaacrylat, Pentaerythrit-Trimethacrylat, Pentaerythrit-Tetramethacrylat, Dipentaerythrit-Tetramethacrylat, Dipentaerythrit-Pentamethacrylat und deren Reaktionsprodukte mit aliphatischen oder aromatischen Diisocyanaten sowie umfassend Dipentaerythrit-Hexaacrylat und Dipentaerythrit-Hexamethacrylat. Bevorzugt handelt es sich bei der Komponente iv) um Mischungen enthaltend zwei oder mehrere der vorangehend genannten Monomere.

Als aliphatische Diisocyanate eignen sich lineare aliphatische, verzweigte aliphatische und/oder cycloaliphatische Diisocyanate. Beispiele für solche aliphatischen Diisocyanate sind 1,4-Butylendiisocyanat, 1,6- Hexamethylendiisocyanat (HDI), Isophorondiisocyanat (IPDI), 2,2,4 und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), Alkyl-2,6-diisocyanatohexanoate (Lysindiisocyanate) mit Alkylgruppen mit 1 bis 8 Kohlenstoffatomen sowie Mischungen aus diesen.

Beispiele für aromatische Diisocyanate sind 1,4-Phenylendiisocyanat, 2,4- und/oder 2,6-Toluylendiisocyanat (TDI), 1,5-Naphthylendiisocyanat, 2,2'- und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI) sowie Mischungen aus diesen.

Bevorzugte aliphatische oder aromatische Diisocyanate sind 1,6-Hexamethylendiisocyanat (HDI), Isophorondiisocyanat (IPDI) oder 2,4- und/oder 2,6-Toluylendiisocyanat (TDI).

In ganz bevorzugten Ausführungsformen enthält die Komponente iv) Pentaerythrit-Triacrylat, Pentaerythrit-Tetraacrylat, Dipentaerythrit-Tetraacrylat, Dipentaerythrit-Pentaacrylat und/oder Dipentaerythrit-Hexaacrylat.

Die Lackzusammensetzung enthält bevorzugt 10 bis 60 Gew.-Teile, besonders bevorzugt 15 bis 55 Gew.-Teile der Komponente v). Bei den genannten Gewichtsteilen handelt es sich um die Summe der Gewichtsteile aller Mono-, Di-, oder Triacrylate oder -methacrylate, aus denen sich die Komponente v) zusammensetzt.

Bei den Monoacrylaten oder -methacrylaten für die Komponente (IV) kann es sich um gegebenenfalls substituierte aliphatische, cycloaliphatische, aromatische, gemischt aromatisch-aliphatische Monoacrylate oder -methacrylate handeln. Dabei kommen sowohl lineare als auch verzeigte aliphatische Monoacrylate oder -methacrylate in Frage, in denen die Alkylkette zudem durch ein oder mehrere Heteroatome, wie z.B. Sauerstoffatome unterbrochen sein kann. Im Falle der cycloaliphatischen oder aromatischen Monoacrylate oder -methacrylate kommen auch heterocyclische oder heteroaromatische Monoacrylate oder -methacrylate in Frage. Die in Frage kommenden Monoacrylate oder -methacrylate für die Komponente (IV) sind nicht alkoxyliert.

Beispiele für solche Monoacrylate oder -methacrylate sind Methylacrylat, Ethylacrylat, n-Butylacrylat, Isobutylacrylate, t-Butylacrylat, 2-Ethyl-hexylacrylat, Isodecylacrylat, n-Laurylacrylat, C12-C15-Alkylacrylate, n-Stearylacrylat, n-Butoxyethylacrylat, Butoxydiethylenglycolacrylat, Methoxytriethylenglycolacrylat, Cyclohexylacrylat, Tetrahydrofurfurylacrylat, Benzylacrylate, 2-Phenoxyethylacrylat, Isobornylacrylat, 2-Hydroxyethylacrylat, 2-Hydroxypropylacrylate, 2-Hydroxyethylacrylate, 2-Hydroxybutylacrylate, 2-Hydroxybutylacrylate und die entsprechenden Methacrylate.

Bei den Diacrylaten oder -methacrylaten für die Komponente (v) kann es sich beispielsweise um solche handeln, die von den Diol-Diacrylaten und -methacrylaten der Komponente ii) verschieden und nicht alkoxyliert sind.

Beispiele für solche Diacrylate oder -methacrylate sind Methandioldiacrylat, Methandioldimethacrylat, Glycerindiacrylat, Glycerindimethacrylat, Neopentylglycoldiacrylat, Neopentylglycoldimethacrylat, 2-Butyl-2-Ethyl-1,3-Propandioldiacrylate, 2-Butyl-2-Ethyl-1,3-Propandioldimethacrylat, Trimethylolpropandiacrylat oder Trimethylolpropandimethacrylat.

Bei den Triacrylaten oder -methacrylaten für die Komponente (v) kann es sich beispielsweise um solche handeln, die von den Triacrylaten und -methacrylaten der Komponente iv) verschieden und nicht alkoxyliert sind.

Beispiele für solche Triacrylate oder -methacrylate sind Glycerintriacrylat, Glycerintrimethacrylat, 1,2,4-Butantrioltriacrylat, 1,2,4-Butantrioltrimethacrylat, Trimethylolpropantriacrylat, Trimethylolpropantrimethacrylat, Tricyclodecandimethanoldiacrylat, Tricyclodecandimethanoldimethacrylat, Ditrimethylolpropantetraacrylat oder Ditrimethylolpropantetramethacrylat.

Komponente (v) enthält bevorzugt wenigstens ein Di- oder Triacrylat oder wenigstens ein Di- oder Trimethacrylat. Komponente (IV) enthält besonders bevorzugt wenigstens ein Triacrylat oder - methacrylat. Besonders bevorzugt enthält Komponente (v) Trimethylolpropantriacrylat und/oder Trimethylolpropantrimethacrylat, vorzugsweise Trimethylolpropantriacrylat.

Geeignete Photoinitiatoren (UV-getriebene Initiatoren) besitzen vorzugsweise eine hohe photochemische Reaktivität und eine Absorptionsbande im nahen UV-Bereich (>300 nm und besonders bevorzugt >350 nm).

Geeignete Photoinitiatoren sind vorzugsweise solche ausgewählt aus der Gruppe aus Acylphosphinoxid-Derivaten, α-Aminoalkylphenon-Derivaten, Hydroxyalkylphenonen Benzophenone, Benzilketale, Methylbenzoylformat und Phenylacetophenonen.

Beispiele für solche Photoinitiatoren sind Benzophenon, Bis(2,4,6-trimethylbenzoyl)phenylphosphinoxid (Irgacure® 819 von BASF AG), 1-Hydroxy-cyclohexyl-phenyl-keton (Irgacure® 184 von BASF AG), 2-Benzyl-2-(dimethylamino)-1-(4-morpholinophenyl)-1-butanon (Irgacure® 369 von BASF AG), 2-Methyl-1-[4-(methylthio)phenyl]-2-morpholino-1-propanon (Irgacure® 907 von BASF AG), (1-Hydroxycyclohexyl)phenylmethanon (Irgacure® 1800 von BASF AG), 2-Hydroxy-2-methyl-1-phenyl-1-propanon (Irgacure® 1700 von BASF AG), Bis(2,6-dimethylbenzoyl)(2,4,4-trimethylpentyl)phosphinoxid, Bis(2,6-dimethoxybenzoyl)(2,4,4-trimethylpentyl)phosphinoxid, (2,4,6-Trimethylbenzoyl)diphenylphosphinoxid (Lucirin®) TPO Solid von der BASF AG), 2,4,6-Trimethylbenzoylethoxyphenylphosphinoxid (Lucirin®) TPO-L von der BASF AG), Benzoylphosphonsäurebis(2,6-dimethylphenyl)ester (Lucirin®) 8728 von der BASF AG), und 2-Hydroxy-2-methyl-1-phenyl-1-propanon (Darocur® 4265 von BASF AG).

Ebenfalls geeignet sind Mischungen dieser Photoinitiatoren untereinander.

Die Lackzusammensetzung kann zudem über die 100 Gew.-Teile der Komponenten i) bis v) hinaus optional ein oder mehrere weitere Lackadditive enthalten. Solche Lackadditive können beispielsweise ausgewählt sein aus der Gruppe enthaltend Stabilisatoren, Verlaufsmittel, Oberflächenadditive, Pigmente, Farbstoffe, anorganische Nanopartikel, Haftvermittler, IR-Absorber, bevorzugt aus der Gruppe enthaltend Stabilisatoren, Verlaufsmittel, Oberflächenadditive und anorganische Nanopartikel. Die Lackzusammensetzung enthält bevorzugt zusätzlich zu der Menge des Photoinitiators und zusätzlich zu den 100 Gew.-Teilen der Komponenten i) bis v) als Komponente vii) 0 bis 20 Gew.-Teile, besonders bevorzugt 0 bis 10 Gew.-Teile, ganz besonders bevorzugt 0,1 bis 10 Gew.-Teile wenigstens eines weiteren Lackadditivs. Bevorzugt beträgt der Gesamtanteil aller in der Lackzusammensetzung enthaltenen Lackadditive 0 bis 20 Gew.-Teile, besonders bevorzugt 0 bis 10 Gew.-Teile, ganz besonders bevorzugt 0,1 bis 10 Gew.-Teile.

Die Lackzusammensetzung kann anorganische Nanopartikel zur Erhöhung der mechanischen Beständigkeit, wie z.B. Kratzfestigkeit und/oder Bleistifthärte enthalten.

Als Nanopartikel kommen anorganische Oxide, Mischoxide, Hydroxide, Sulfate, Carbonate, Carbide, Boride und Nitride von Elementen der II bis IV Hauptgruppe und/oder Elementen der I bis VIII Nebengruppe des Periodensystems einschließlich der Lanthanide in Frage. Bevorzugte Nanopartikel sind Siliziumoxid-, Aluminiumoxid-, Ceroxid-, Zirkonoxid-, Nioboxid-, Zinkoxid- oder Titanoxid-Nanopartikel, besonders bevorzugt sind Siliziumoxid-Nanopartikel.

Die eingesetzten Partikel weisen vorzugsweise mittlere Partikelgrößen (gemessen mittels dynamischer Lichtstreuung in Dispersion bestimmt als Z-Mittelwert) kleiner 200 nm, bevorzugt von 5 bis 100 nm, besonders bevorzugt 5 bis 50 nm auf. Bevorzugt weisen wenigstens 75%, besonders bevorzugt wenigstens 90 %, ganz besonders bevorzugt wenigstens 95% aller eingesetzten Nanopartikel die vorstehend definierten Größen auf.

Die Lackzusammensetzung kann zudem über die 100 Gew.-Teile der Komponenten i) bis v) hinaus optional ein oder mehrere organische Lösungsmittel enthalten. Solche organischen Lösungsmittel können beispielsweise ausgewählt sein aus der Gruppe enthaltend aromatische Lösemittel, wie z.B. Xylol oder Toluol, Ketone, wie z.B. Aceton, 2-Butanon, Methyl-isobutylketon, Diacetonalkohol, Alkohole, wie z.B. Methanol, Ethanol, i-Propanol, Butanol,2-Methoxy-propylalkohol, Ether, wie z.B. 1,4-Dioxan, Ethylenglykol-n-propylether, oder Ester, wie z.B. Essigsäureethylester, Essigsäurebutylester, 1-Methoxy-2-propylacetat oder Mischungen enthaltend diese Lösungsmittel. Besonders bevorzugt sind Ethanol, i-Propanol, Butanol, Essigsäureethylester, Essigsäurebutylester, 2-Methoxy-propylalkohol, Diacetonalkohol, Xylol oder Toluol. Ganz besonders bevorzugt sind i-Propanol, Butanol, Essigsäureethylester, Essigsäurebutylester, 2-Methoxy-propylalkohol. Die Lackzusammensetzung enthält bevorzugt zusätzlich zu der Menge des Photoinitiators und zusätzlich zu den 100 Gew.-Teilen der Komponenten i) bis v) als Komponente viii) 0 bis 300 Gew.-Teile, besonders bevorzugt 0 bis 200 Gew.-Teile, ganz besonders bevorzugt 10 bis 150 Gew.-Teile wenigstens eines organischen Lösungsmittels. Bevorzugt beträgt der Gesamtanteil aller in der Lackzusammensetzung enthaltenen organischen Lösungsmittel 0 bis 300 Gew.-Teile, besonders bevorzugt 0 bis 200 Gew.-Teile, ganz besonders bevorzugt 10 bis 150 Gew.-Teile.

Die Lackzusammensetzungen können auf einfache Weise hergestellt werden, indem die einzelnen Komponenten i) bis vi) und gegebenenfalls die optionalen Komponenten vii) und viii) entweder in Abwesenheit von Lösungsmittel(n) zusammen gegeben und durch Rühren miteinander vermischt werden oder in Anwesenheit von Lösungsmittel(n) beispielsweise in das oder die Lösungsmittel gegeben und durch Rühren miteinander vermischt werden. Bevorzugt wird zuerst der Photoinitiator in dem oder den Lösungsmitteln oder beispielsweise in der Komponente I) gelöst und anschließend die weiteren Komponenten hinzugegeben. Gegebenenfalls erfolgt anschließend noch eine Reinigung mittels Filtration, vorzugsweise mittels Feinfiltration.

Die flüssige Form des UV-Absorbers (Formel I, I-1, I-2) macht die Zumischung der Komponenten i) bis vi) besonders leicht und die angepasste Struktur des Absorbers macht den entstehenden fertigen Lack stabil und transparent in allen Schritten des Beschichtungsprozesses und dadurch besonders geeignet für die Herstellung der UV-schützenden, witterungsbeständigen, klaren und harten Oberfläche eines Kunststoffteils oder einer Folie (besonders aus Polycarbonat).

Ein weiterer Gegenstand der vorliegenden Erfindung ist ein Verfahren zum Beschichten eines Substrats, umfassend die Schritte:
- Auftragen einer erfindungsgemäßen Beschichtungszusammensetzung auf ein Substrat
- Aushärten der zuvor aufgetragenen Beschichtungszusammensetzung durch Bestrahlen mit UV-Licht.

Vorzugsweise wird im ersten Schritt die Zusammensetzung auf die Oberfläche des Substrats durch Fluten, Tauchen, Sprühen, Aufwalzen oder Aufschleudern aufgebracht und anschließend bei Raumtemperatur und/oder erhöhter Temperatur (vorzugsweise bei 20 - 200 °C, besonders bevorzugt bei 40 - 120 °C) abgelüftet. Die Oberfläche der zweiten Schicht kann durch Reinigung oder Aktivierung vorbehandelt sein.

Vorzugsweise erfolgt im zweiten Schritt (ii) das Härten der ersten Schicht mittels UV-Licht, wobei als UV-Licht Quelle bevorzugt eine Quecksilberdampflampe, dotiert mit Eisen, oder auch eine reine Quecksilberdampflampe bzw. eine mit Gallium dotierte dienen. Auf diese Weise wird mit Licht einer Wellenlänge von 254 nm bestrahlt.

Durch die erfindungsgemäße Dosis von mindestens 3 J/cm² erreicht man eine Durchhärtung der gesamten Schicht des Beschichtungsmittels und ein Einbau des UV-Absorbers in die sich bildende Polymermatrix.

In einer Ausführungsform des erfindungsgemäßen Verfahrens ist das Substrat ein thermoplastisches Substrat.

In einer weiteren Ausführungsform des erfindungsgemäßen Verfahrens ist das Substrat ein Polycarbonatsubstrat.

### Substrate

Thermoplastische Polymere des Substrats im erfindungsgemäßen Sinne sind Polycarbonat, Polyestercarbonat, Polyester (wie beispielsweise Polyalkylenterephthalat), Polymethylmethacrylat Polyphenylenether, Pfropfcopolymere (wie beispielsweise ABS) und deren Mischungen.

Die zweite Schicht ist bevorzugt Polycarbonat, insbesondere Homopolycarbonat, Copolycarbonat und/oder thermoplastisches Polyestercarbonat.

Sie haben bevorzugt mittlere Molekulargewichte M̅_{w} von 18.000 bis 40.000, vorzugsweise von 22.000 bis 36.000 und insbesondere von 24.000 bis 33.000, ermittelt durch Messung der relativen Lösungsviskosität in Dichlormethan oder in Mischungen gleicher Gewichtsmengen Phenol/o-Dichlorbenzol geeicht durch Lichtstreuung.

Den erfindungsgemäßen Polycarbonaten sowie den ggf. weiteren enthaltenen -Stabilisatoren, Thermostabilisatoren, Antistatika und Pigmente in den üblichen Mengen zugesetzt werden; gegebenenfalls können das Entformungsverhalten und/oder das Fließverhalten, noch durch Zusatz externer Entformungsmittel und/oder Fließmittel, verbessert werden (z. B. Alkyl- und Arylphosphite, -phosphate, -phosphane, -niedermolekulare Carbonsäureester, Halogenverbindungen, Salze, Kreide, Quarzmehl, Glas- und Kohlenstofffasern, Pigmente und deren Kombination). Solche Verbindungen werden z. B. in WO 99/55772, S. 15 - 25, EP 1 308 084 und in den entsprechenden Kapiteln des "Plastics Additives Handbook", ed. Hans Zweifel, 5^{th} Edition 2000, Hanser Publishers, Munich beschrieben.

Zur Herstellung von Polycarbonaten sei beispielhaft auf WO 2004/063249 A1, WO 2001/05866 A1, WO 2000/105867, US-A 5,340,905, US 5,097,002, US-A 5,717,057 und der dort zitierten Literatur hingewiesen.

Die Herstellung der Polycarbonate erfolgt vorzugsweise nach dem Phasengrenzflächenverfahren oder dem Schmelze-Umesterungsverfahren und wird im Folgenden beispielhaft an dem Phasengrenzflächenverfahren beschrieben.

Als Ausgangsverbindungen bevorzugt einzusetzende Verbindungen sind Bisphenole der allgemeinen Formel HO-R-OH, worin R ein divalenter organischer Rest mit 6 bis 30 Kohlenstoffatomen ist, der eine oder mehrere aromatische Gruppen enthält.

Beispiele solcher Verbindungen sind Bisphenole, die zu der Gruppe der Dihydroxydiphenyle, Bis(hydroxyphenyl)alkane, Indanbisphenole, Bis(hydroxyphenyl)ether, Bis(hydroxyphenyl)-sulfone, Bis(hydroxyphenyl)ketone und α,α'-Bis(hydroxyphenyl)-diisopropylbenzole gehören.

Besonders bevorzugte Bisphenole, die zu den vorgenannten Verbindungsgruppen gehören, sind Bisphenol-A, Tetraalkylbisphenol-A, 4,4-(meta-Phenylendiisopropyl)diphenol (Bisphenol M), 4,4-(para-Phenylendiisopropyl)diphenol, 1,1-Bis-(4-hydroxyphenyl)-3,3,5-trimethylcyclohexan (BP-TMC) sowie gegebenenfalls deren Gemische.

Bevorzugt werden die erfindungsgemäß einzusetzenden Bisphenolverbindungen mit Kohlensäureverbindungen, insbesondere Phosgen, oder beim Schmelzeumesterungsprozess mit Diphenylcarbonat bzw. Dimethylcarbonat, umgesetzt.

Polyestercarbonate werden bevorzugt durch Umsetzung der bereits genannten Bisphenole, mindestens einer aromatischen Dicarbonsäure und gegebenenfalls Kohlensäureäquivalente erhalten. Geeignete aromatische Dicarbonsäuren sind beispielsweise Phthalsäure, Terephthalsäure, Isophthalsäure, 3,3'- oder 4,4'-Diphenyldicarbonsäure und Benzophenondicarbonsäuren. Ein Teil, bis zu 80 Mol.-%, vorzugsweise von 20 bis 50 Mol-% der Carbonatgruppen in den Polycarbonaten können durch aromatische Dicarbonsäureester-Gruppen ersetzt sein.

Beim Phasengrenzflächenverfahren verwendete inerte organische Lösungsmittel sind beispielsweise Dichlormethan, die verschiedenen Dichlorethane und Chlorpropanverbindungen, Tetrachlormethan, Trichlormethan, Chlorbenzol und Chlortoluol, vorzugsweise werden Chlorbenzol oder Dichlormethan bzw. Gemische aus Dichlormethan und Chlorbenzol eingesetzt.

Die Phasengrenzflächenreaktion kann durch Katalysatoren wie tertiäre Amine, insbesondere N-Alkylpiperidine oder Oniumsalze beschleunigt werden. Bevorzugt werden Tributylamin, Triethylamin und N-Ethylpiperidin verwendet. Im Falle des Schmelzeumesterungsprozesses werden bevorzugt die in DE-A 4 238 123 genannten Katalysatoren verwendet.

Die Polycarbonate können durch den Einsatz geringer Mengen Verzweiger bewusst und kontrolliert verzweigt werden. Einige geeignete Verzweiger sind: Phloroglucin, 4,6-Dimethyl-2,4,6-tri-(4-hydroxyphenyl)-hepten-2; 4,6-Dimethyl-2,4,6-tri-(4-hydroxyphenyl)-heptan; 1,3,5-Tri-(4-hydroxyphenyl)-benzol; 1,1,1-Tri-(4-hydroxyphenyl)-ethan; Tri-(4-hydroxyphenyl)-phenylmethan; 2,2-Bis-[4,4-bis-(4-hydroxyphenyl)-cyclohexyl]-propan; 2,4-Bis-(4-hydroxyphenyl-isopropyl)-phenol; 2,6-Bis-(2-hydroxy-5'-methyl-benzyl)-4-methylphenol; 2-(4-Hydroxyphenyl)-2-(2,4-dihydroxyphenyl)-propan; Hexa-(4-(4-hydroxyphenyl-isopropyl)-phenyl)-orthoterephthalsäureester; Tetra-(4-hydroxyphenyl)-methan; Tetra-(4-(4-hydroxyphenyl-isopropyl)-phenoxy)-methan; α,α,'α"-Tris-(4-hydroxyphenyl)-1,3,5-triisopropylbenzol; 2,4-Dihydroxybenzoesäure; Trimesinsäure; Cyanurchlorid; 3,3-Bis-(3-methyl-4-hydroxyphenyl)-2-oxo-2,3-dihydroindol; 1,4-Bis-(4',4"-dihydroxytriphenyl)-methyl)-benzol und insbesondere: 1,1,1-Tri-(4-hydroxyphenyl)-ethan und Bis-(3-methyl-4-hydroxyphenyl)-2-oxo-2,3-dihydroindol.

Die gegebenenfalls mitzuverwendenden 0,05 bis 2 Mol-%, bezogen auf eingesetzte Diphenole, an Verzweigern bzw. Mischungen der Verzweigern, können mit den Diphenolen zusammen eingesetzt werden aber auch in einem späteren Stadium der Synthese zugegeben werden.

Als Kettenabbrecher werden bevorzugt Phenole wie Phenol, Alkylphenole wie Kresol und 4-tert.-Butylphenol, Chlorphenol, Bromphenol, Cumylphenol oder deren Mischungen verwendet in Mengen von 1 - 20 Mol-%, bevorzugt 2 - 10 Mol-% je Mol Bisphenol. Bevorzugt sind Phenol, 4-tert.-Butylphenol bzw. Cumylphenol.

Kettenabbrecher und Verzweiger können getrennt oder aber auch zusammen mit dem Bisphenol den Synthesen zugesetzt werden.

Die Herstellung der Polycarbonate nach dem Schmelzeumesterungsprozess ist in DE-A 4238 123 beispielhaft beschrieben.

Erfindungsgemäß bevorzugte Polycarbonate für die zweite Schicht des erfindungsgemäßen mehrschichtigen Erzeugnisses sind das Homopolycarbonat auf Basis von Bisphenol A, das Homopolycarbonat auf Basis von 1,1-Bis-(4-hydroxyphenyl)-3,3,5-trimethylcyclohexan und die Copolycarbonate auf Basis der beiden Monomere Bisphenol A und 1,1-Bis-(4-hydroxyphenyl)-3,3,5-trimethylcyclohexan.

Das Homopolycarbonat auf Basis von Bisphenol A ist besonders bevorzugt.

Das Polycarbonat kann Stabilisatoren enthalten. Geeignete Stabilisatoren sind beispielsweise Phosphine, Phosphite oder Si enthaltende Stabilisatoren und weitere in EP-A 0 500 496 beschriebene Verbindungen. Beispielhaft seien Triphenylphosphite, Diphenylalkylphosphite, Phenyldialkylphosphite, Tris-(nonylphenyl)phosphit, Tetrakis-(2,4-di-tert.-butylphenyl)-4,4'-biphenylen-diphosponit und Triarylphosphit genannt. Besonders bevorzugt sind Triphenylphosphin und Tris-(2,4-di-tert.-butylphenyl)phosphit.

Ferner kann das Polycarbonat enthaltende Substrat des erfindungsgemäßen mehrschichtigen Erzeugnisses 0,01 bis 0,5 Gew.-% der Ester oder Teilester von ein- bis sechswertigen Alkoholen, insbesondere des Glycerins, des Pentaerythrits oder von Guerbetalkoholen enthalten.

Einwertige Alkohole sind beispielsweise Stearylalkohol, Palmitylalkohol und Guerbetalkohole.

Ein zweiwertiger Alkohol ist beispielsweise Glycol.

Ein dreiwertiger Alkohol ist beispielsweise Glycerin.

Vierwertige Alkohole sind beispielsweise Pentaerythrit und Mesoerythrit.

Fünfwertige Alkohole sind beispielsweise Arabit, Ribit und Xylit.

Sechswertige Alkohole sind beispielsweise Mannit, Glucit (Sorbit) und Dulcit.

Die Ester sind bevorzugt die Monoester, Diester, Triester, Tetraester, Pentaester und Hexaester oder deren Mischungen, insbesondere statistische Mischungen, aus gesättigten, aliphatischen C₁₀ bis C₃₆-Monocarbonsäuren und gegebenenfalls Hydroxy-Monocarbonsäuren, vorzugsweise mit gesättigten, aliphatischen C₁₄ bis C₃₂-Monocarbonsäuren und gegebenenfalls Hydroxy-Monocarbonsäuren.

Die kommerziell erhältlichen Fettsäureester, insbesondere des Pentaerythrits und des Glycerins, können herstellungsbedingt <60 % unterschiedlicher Teilester enthalten.

Gesättigte, aliphatische Monocarbonsäuren mit 10 bis 36 C-Atomen sind beispielsweise Caprinsäure, Laurinsäure, Myristinsäure, Palmitinsäure, Stearinsäure, Hydroxystearinsäure, Arachinsäure, Behensäure, Lignocerinsäure, Cerotinsäure und Montansäuren.

Bevorzugte gesättigte, aliphatische Monocarbonsäuren mit 14 bis 22 C-Atomen sind beispielsweise Myristinsäure, Palmitinsäure, Stearinsäure, Hydroxystearinsäure, Arachinsäure und Behensäure.

Besonders bevorzugt sind gesättigte, aliphatische Monocarbonsäuren wie Palmitinsäure, Stearinsäure und Hydroxystearinsäure.

Die gesättigten, aliphatischen C₁₀ bis C₃₆-Carbonsäuren und die Fettsäureester sind als solche entweder literaturbekannt oder nach literaturbekannten Verfahren herstellbar. Beispiele für Pentaerythritfettsäureester sind die der besonders bevorzugten, vorstehend genannten Monocarbonsäuren. Besonders bevorzugt sind Ester des Pentaerythrits und des Glycerins mit Stearinsäure und Palmitinsäure. Besonders bevorzugt sind auch Ester von Guerbetalkoholen und des Glycerins mit Stearinsäure und Palmitinsäure und gegebenenfalls Hydroxystearinsäure.

Die Erfindung umfasst auch ein Mehrschichtaufbau enthaltend das Substrat A und eine Schutzschicht B hergestellt durch Aushärten der Zusammensetzung entsprechend der Erfindung. Optional sind weitere Schichten möglich, entweder auf der gehärteten Zusammensetzung oder auf dem Substrat bevor die Zusammensatzung entsprechend der Erfindung aufgetragen wird. Ebenso sind weitere Schichten, möglich mit einer Schichtabfolge B-A-B. Die Schichten B können dabei identisch oder unterschiedlich nach der beschriebenen Zusammensetzung sein.

Die erfindungsgemäßen mehrschichtigen Erzeugnisse bzw. die zur Herstellung verwendeten thermoplastischen Polymere können organische Farbstoffe, anorganische Farbpigmente, Fluoreszenzfarbstoffe und besonders bevorzugt optische Aufheller enthalten.

Weiterhin betrifft die vorliegende Erfindung ein beschichtetes Substrat, erhältlich durch ein erfindungsgemäßes Verfahren.

### Beispiele

Die Erfindung wird anhand der nachfolgenden Beispiele weiter beschrieben, ohne jedoch darauf beschränkt zu sein.

### Beispiel 1

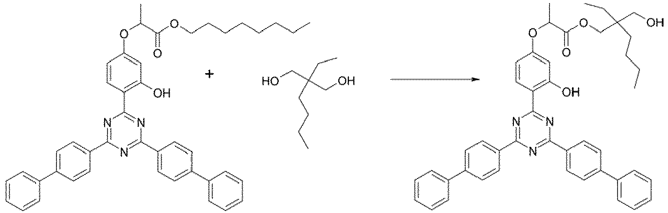

400 g Tinuvin® 479 (BASF), 945 g 2-Butyl-2-ethyl-1,3-propandiol (Aldrich) und 29 g Dibutylzinnoxid (Aldrich) wurden eingewogen, zusammengebracht und 5 Std. bei 155 °C gerührt. Anschließend wurde das gebildete Octanol im Vakuum von 10 bis 20 mbar abdestilliert. Ansatz wurde abgekühlt und in 2500 ml Methanol einrührt. Niederschlag wurde abfiltriert und im Vakuum getrocknet. Der Feststoff wurde in 700 ml einer Mischung von Toluol/Ethylacetat (8:1) gelöst. Die Lösung wurde durch eine Schicht (10 cm stark) von Kieselgel filtriert. Das Filtrat wurde eindampft. Der Feststoff wurde in Methanol suspendiert, auf den Filter gebracht und dann in Vakuum bei 40 °C getrocknet. Dieses Produkt wird in den nachfolgenden Beispielen auch als "Zwischenprodukt" der Formel (IV-4) bezeichnet.
Ausbeute 366 g (88% d.Th.)
Schmelzpunkt 80,2°C
Elementaranalyse: C₄₅H₄₅N₃O₅ (707,88)
Ber.: C76,36; H6,41; N5,94.
Gef.: C76,00; H6,40; N5,90.

### Beispiel 2

Analog zu Beispiel 1 wurde aus 153 g Tinuvin® 479 und 300 g 2,2-Diethyl-1,3-propandiol 125 g (80% d.Th) des Zwischenprodukts der Formel (IV-3) hergestellt. 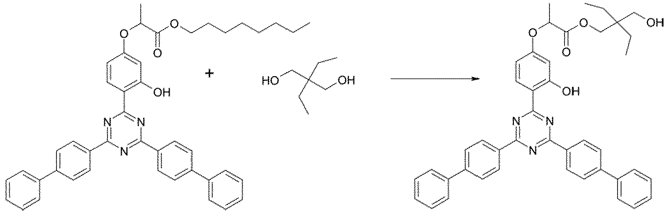 Schmelzpunkt 107°C
Elementaranalyse: C₄₃H₄₁N₃O₅ (679,82)
Ber.: C75,97; H6,08; N6,18.
Gef.: C75,60; H6,20; N6,20.

### Beispiel 3

Analog zu Beispiel 1 wurde aus 400 g Tinuvin® 479 und 532 g 2-Methyl-1,3-propandiol 294 g (78% d.Th) des Zwischenprodukts der Formel (IV-1) hergestellt. 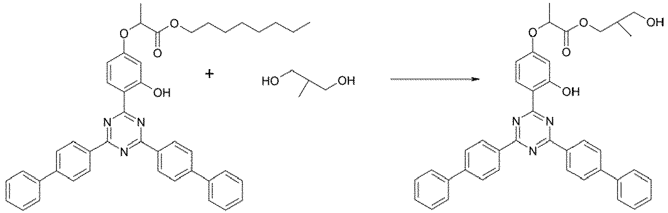 Schmelzpunkt 153°C
Elementaranalyse: C₄₀H₃₅N₃O₅ (637,74)
Ber.: C75,34; H5,53; N6,59.
Gef.: C75,30; H5,70; N6,50.

### Beispiel 4

Analog zu Beispiel 1 wurden aus 899,9 g Tinuvin® 479 und 1382,6 g 2,2-Dimethyl-1,3-propandiol 722,7 g (83,5% d.Th) des Zwischenprodukts der Formel (IV-2) hergestellt. 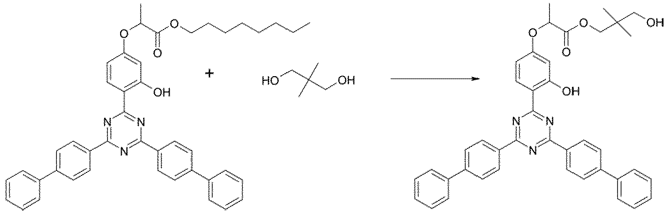 Schmelzpunkt 173°C

### Beispiel 5

Desmolux® D100 (Fa. Bayer MaterialScience) ist ein isocyanatgruppenhaltiges Urethanacrylat, Reaktiwerdünner-frei, mit einer Viskosität (23 °C) von 10000 ± 2500 mPas und einem NCO-Gehalt von 12,8±1%.

6,8 g des aus Beispiel 2 erhaltenen Zwischenprodukts wurden in 11,61 g 2-Hydroxyethylacrylat bei 100 °C gelöst. Die Lösung wurde bis 65 °C abgekühlt und dem bis auf 65 °C vorgeheizten Desmolux® D100 (66 g) unter Rühren zugegeben. Das Reaktionsgemisch wurde 5h bei 65°C weiter gerührt. Weitere 10,5g 2-Hydroxyethylacrylat wurden dem Reaktionsgemisch zugegeben und man ließ für 2h abreagieren. Die Heizung wurde abgestellt. 40,65 g Isopropanol wurden zugegeben und eingerührt. Eine klare hellgelbe mittelviskose Flüssigkeit entstand. Der NCO-Restgehalt betrug <0,1%, der Feststoffgehalt 70 Gew.-%. Der effektive UV-Absorber-Gehalt (Rest der Substanz aus Beispiel 2 im Harz betrug 7,5 Gew.-% (fest/fest).

### Beispiel 6

28,55 g des aus Beispiel 2 erhaltenen Zwischenprodukts wurden in eine Mischung aus 50 g Butylacetat und 11,61 g 2-Hydroxyethylacrylat bei 100 °C gelöst. Die Lösung wurde bis 65 °C abgekühlt und dem bis auf 65 °C vorgeheizten Desmolux® D100 (66 g) unter Rühren zugegeben. Das Reaktionsgemisch wurde für 6h bei 65 °C weiter gerührt. Weitere 6,8g 2-Hydroxyethylacrylat wurden dem Reaktionsgemisch zugegeben und man ließ für 2h abreagieren. Butylacetat wurde im Vakuum (1-2 mbar) und einer Badtemperatur von 40 °C komplett abdestilliert. 48,5g Isopropanol wurden zugegeben und bei 65 °C eingerührt. Man erhielt eine klare gelbe mittelviskose Flüssigkeit (Produkt des Beispiels 6) mit einem NCO-Restgehalt von <0,1%, einer Viskosität (23 °C) von 282 mPas und einem Feststoffgehalt von 70 Gew.-%. Der effektive UV-Absorber-Gehalt (Rest der Substanz aus Beispiel 2) im Harz betrug 25 Gew.-% (fest/fest).

### Beispiel 7

68 g des aus Beispiel 2 erhaltenen Zwischenprodukts wurden in 150 g Butylacetat bei 100 °C gelöst. Die Lösung wurde bis 90 °C abgekühlt und dem bis auf 90 °C vorgeheizten Desmolux® D100 (66 g) unter Rühren zugegeben. Das Reaktionsgemisch wurde für 8h bei 90 °C weiter gerührt und dann bis 65 °C abgekühlt. 11,7g 2-Hydroxyethylacrylat wurden dem Reaktionsgemisch zugegeben und man ließ für 2h abreagieren. Butylacetat wurde im Vakuum (1-2 mbar) und einer Badtemperatur von 60 bis 80 °C komplett abdestilliert. Man erhielt eine klare gelb-orange, sehr viskose (feste bei Raumtemperatur) Flüssigkeit (Produkt des Beispiels 7) mit einem NCO-Restgehalt von <0,1%, einer Viskosität (80 °C) von 60000 mPas und einem Feststoffgehalt von 100 Gew.-%. Der effektive UV-Absorber-Gehalt (Rest der Substanz aus Beispiel 2) im Harz betrug 46 Gew.-% (fest/fest).

70 g Harz (Produkt des Beispiels 7) werden 30 g 1-Methoxy-2-propanol bei 100°C zugemischt. Man erhält eine klare gelbe viskose Flüssigkeit mit einer Viskosität (23 °C) von 2110 mPas und Feststoffgehalt von 70 Gew.-%.

### Beispiel 8

2101,3 g des aus Beispiel 4 erhaltenen Zwischenprodukts wurden in 3877,6 g Diacetonalkohol bei 130 °C gelöst. Die Lösung wurde auf 80 °C abgekühlt, durch einen Filter T1000 (Fa. Seitz) filtriert und dem bis auf 90 °C vorgeheizten Desmolux® D100 (5280,0 g) beim Rühren zugegeben. Das Reaktionsgemisch wurde für 4h bei 90 °C weiter gerührt und dann bis 80 °C abgekühlt. Der NCO-Gehalt wurde bestimmt. Dann wurde dem Reaktionsgemisch die berechnete Menge von 862,0 g von 2-Hydroxyethylacrylates zugegeben und man ließ für 8h abreagieren. Danach wurde Ansatz abgestellt, abgekühlt und durch einen T5500 Filter (Fa. Seitz) in bereitstehende Fässer gepresst.
Ausbeute: 11568 g
NCO-Gehalt des Produktes: <0,1%; Gehalt Zinn: < 1 mg/kg

Analytische Gelpermeationschromatographie: M_{w}=2,06 *10³; Mₙ=1,02*10³. Gewichtsanteil der Moleküle mit Molmasse zwischen 500 und 800 g/mol: 2,4%. Das zeigt, dass das aus Beispiel 4 erhaltene Zwischenprodukt größtenteils abreagiert hat und an Urethanacrylat gebunden ist. Festkörpergehalt des Produktes (nach 2 h bei 140 °C): 70,4%; Viskosität (23 °C): 8480 mPas.

### Beispiel 9

21,5 g des aus Beispiel 1 erhaltenen Zwischenprodukts wurden als Pulver portionsweise dem bis auf 95 °C vorgeheizten Desmolux®) D100 (66 g) unter Rühren zugegeben. Das Reaktionsgemisch wurde 4h bei 95°C weiter gerührt und dann bis 80 °C abgekühlt. 19,8g 2-Hydroxyethylacrylat wurden dem Reaktionsgemisch zugegeben und man ließ für 2h abreagieren. Die Heizung wurde abgestellt. 45,9 g Isopropanol wurden dem klaren gelben flüssigen Harz zugegeben und eingerührt. Eine klare hellgelbe mittelviskose Flüssigkeit entstand. NCO-Restgehalt: <0,1%, Viskosität (23 °C): 618 mPas, Feststoffgehalt: 70 Gew.-%. Der effektive UV-Absorber-Gehalt (Rest der Substanz aus Beispiel 4) im Harz betrug 20 Gew.-% (fest/fest).

### Beispiel 10

28,55 g des aus Beispiel 1 erhaltenen Zwischenprodukts wurden als Pulver portionsweise dem bis auf 95 °C vorgeheizten Desmolux® D100 (66 g) beim Rühren zugegeben. Das Reaktionsgemisch wurde 4h bei 95°C weiter gerührt und dann bis 80 °C abgekühlt. 18,7g 2-Hydroxyethylacrylat wurden dem Reaktionsgemisch zugegeben und man ließ für 2h abreagieren. Die Heizung wurde abgestellt. 48,5 g 1-Methoxy-2-propanol wurden dem klaren gelben flüssigen Harz zugegeben und eingerührt. Eine klare hellgelbe mittelviskose Flüssigkeit entstand. NCO-Restgehalt: <0,1%, Viskosität (23 °C): 1360 mPas, Feststoffgehalt: 70 Gew.-%. Der effektive UV-Absorber-Gehalt (Rest der Substanz aus Beispiel 4) im Harz betrug 25 Gew.-% (fest auf fest).

### Beispiel 11

Für die Herstellung einer kratzfesten Beschichtung wurde folgende Lackzusammensetzung ("Grundlack G") als Grundlack benutzt. Die Gewichtsanteile der Komponenten betrugen:
1,6-Hexandioldiacrylat (HDDA) 48,8;
Trimethylolpropantriacrylat (TMPTA) 20,7;
Ethoxyliertes TMPTA 20,7;
Pentaerythrit-Tetraacrylat (PTTA) 9,8
Summe: 100 Gewichts-Teile.

Für die Herstellung eines erfindungsgemäßen Beschichtungsmittels wurden folgende Komponenten zusammengebracht:

| | |
|---|---|
| UV-Absorber (Produkt aus Beispiel 9; 70% Feststoff in Isopropanol) | 6,00 |
| Grundlack G | 22,40 |
| Isopropanol | 26,20 |
| Irgacure 184 (BASF) | 1,20 |
| Darocur 4265 (BASF) | 0,06 |
| Tinuvin 123 (BASF) | 0,14 |

Summe: 56 Gewichts-Teile. Das Beschichtungsmittel hatte einen Feststoffgehalt von 50% und eine Viskosität (23 °C) von weniger als 10 mPas.

### Beispiel 12

Für die Herstellung eines erfindungsgemäßen Beschichtungsmittels wurden folgende Komponenten zusammengebracht:

| | |
|---|---|
| UV-Absorber (Produkt aus Beispiel 10; 70% Feststoff in 1-Methoxy-2-propanol) | 4,80 |
| Grundlack G (Beispiel 11) | 23,24 |
| 1-Methoxy-2-propanol | 26,56 |
| Irgacure 184 (BASF) | 1,20 |
| Darocur 4265 (BASF) | 0,06 |
| Tinuvin 123 (BASF) | 0,14 |

Summe: 56 Gew.-Teile. Das Beschichtungsmittel hatte einen Feststoffgehalt von 50% und eine Viskosität (23 °C) von weniger als 10 mPas.

### Beispiel 13 (Vergleichsbeispiel)

Für die Herstellung eines Vergleichs-Beschichtungsmittels wurden folgende Komponenten zusammengebracht:

| | |
|---|---|
| Grundlack G (Beispiel 11) | 26,60 |
| 1-Methoxy-2-propanol | 28,00 |
| Irgacure 184 (BASF) | 1,20 |
| Darocur 4265 (BASF) | 0,06 |
| Tinuvin 123 (BASF) | 0,14 |

Summe: 56 Gew.-Teile. Das Beschichtungsmittel hatte einen Feststoffgehalt von 50% und eine Viskosität (23 °C) von weniger als 10 mPas.

### Beispiel 14

Einer beidseitig mit Kaschierfolien bestückten Polycarbonat (PC)-Folie (Makrofol^{®} DE 1-1 cc, Dicke 500 µm) wurden beidseitig die Kaschierfolien abgenommen. Die Folien wurden ohne nasse Reinigung und ohne thermische Vorbehandlung beschichtet.

Die flüssigen Lackformulierungen aus Beispielen 11, 12 und 13 wurden auf die Folien mit Hilfe des Filmziehgerätes Zehntner ZAA 2300 (Universalrakel, Ziehgeschwindigkeit 30 mm/s) appliziert. Die Beschichtungen wurden bei 60 °C für 10 min angetrocknet und mit einem Quecksilberstrahler (Leistung 80 W/cm Lampenlänge) mit einer Dosis von ca. 3000 mJ/cm2 gehärtet.

Die Schichtdicke der Beschichtungen wurde durch Beobachtung der Schnittkante in optischem Mikroskop gemessen. Methode - Auflicht, Hellfeld, Vergrößerung 500x.

Die Extinktion der erfindungsgemäßen Beschichtung nach Applikation auf eine Polycarbonatfolie und anschließender Härtung wurde mit Hilfe eines Cary 50 UV-Vis Spektrophotometers von Varian Inc., USA bei 350 nm bestimmt, wobei ein unbeschichtete, aber sonst identisches Polycarbonatfolie als Hintergrundspektrum verwendet wurde.

Die Bleistifthärte nach ISO 15184, Haftung nach Gitterschnittprüfung (ISO 2409) sowie die Lösemittelbeständigkeit gegen Butanol, Methylethylketon, Ethylacetat und N-Ethylpyrrolidon (1 Stunde; in Anlehnung an EN ISO 2812-3:2007) wurden bestimmt.

| | **Beispiel 11** | **Beispiel 12** | **Beispiel 13** |
|---|---|---|---|
| Schichtdicke (µm) | 10 | 20 | 10 |
| Optische Dichte (350 nm) | 2,61 | 3,19 | 0,02 |
| Haftung (Gitterschnitt) | 0 (OK) | 0 (OK) | 0 (OK) |
| Haftung (nach 4 Stunden in kochendem Wasser) | 0 (OK) | 0 (OK) | 0 (OK) |
| Lösemittelbeständigkeit (in o.g. Lösemitteln, 1 Stunde) | alle 0 (OK) | alle 0 (OK) | alle 0 (OK) |
| Bleistifthärte | HB | HB | HB |

Die Werte zeigen, dass durch die erfindungsgemäße Einführung der UV-Absorber eine hohe Absorptionskraft in UV-Bereich erzeugt werden kann. Trotzdem beeinflussen die erfindungsgemäßen UV-Absorber keinerlei Haftung, Lösemittelbeständigkeit und Härte der Beschichtung.

### Beispiel 15

Um die UV-schützende Wirkung der UV-Absorber enthaltenden Beschichtung auf das Substrat zu prüfen, wurden die Proben einem Schnellbewitterungstest SAE J 2412 unterzogen. Beanspruchungsdauer: bis zu einer Strahlungsdosis von 64000 kJ/m². Die Farbänderung der Probe wurde in L*, a*, b* Koordinaten gemessen und als Gesamtfarbabstand ΔE* dargestellt.

| | **Beispiel 11** | **Beispiel 12** | **Beispiel 13** |
|---|---|---|---|
| Farbwerte L*, a*, b* vor Bestrahlung | 95,1; -0,7; 2,2 | 95,2; -0,7; 2,2 | 95,0; -0,7; 2,3 |
| Farbwerte L*, a*, b* nach 64000 kJ/m² | 94,9; -0,8; 2,4 | 95,0; -0,8; 2,4 | 93,9; -2,5; 9,9 |
| Gesamtfarbabstand ΔE* durch Bewitterung | 0,3 | 0,3 | 7,9 |

Die Werte zeigen, dass die erfindungsgemäßen UV-Absorber, eingeführt in eine kratzfeste Beschichtung, nur minimale Farbänderung des Substrates durch Bewitterung zulässt. Ungeschützte Substrate vergilben dabei stark.

## Patentansprüche

1. UV-Absorber enthaltendes Urethanacrylat der Formel (I): 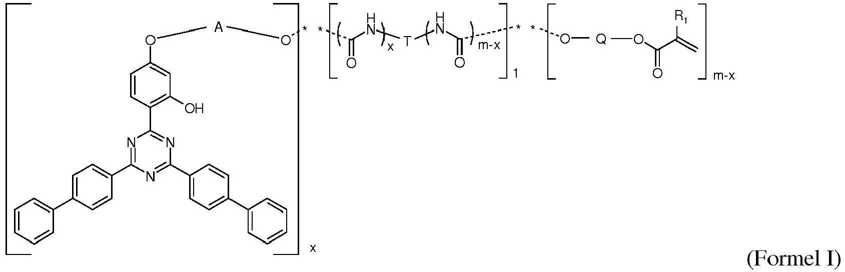 wobei
***R₁*** ein Wasserstoff oder ein Methylrest ist,
***Q*** ein Linker des handelsüblichen Hydroxyalkyl(Meth)acrylates aus der Gruppe von 2-Hydroxyethyl(meth)acrylat, 2-Hydroxypropyl(meth)acrylat, 4-Hydroxybutyl(meth)acrylat, 3-Hydroxy-2,2-dimethylpropyl(meth)acrylat ist,
**T** ein Kern der handelsüblichen aliphatischen und cycloaliphatischen Polyisocyanate T(NCO)*ₘ* ist, die cyclische Isocyanurat-, Uretdion-, Iminooxadiazindion- oder Oxadiazintrion-Strukturen aufweisen, ebenso wie verzweigte Biuret-Strukturen in Falle der cycloaliphatischen Polyisocyanate aufweisen.
***m*** dabei die ursprüngliche durchschnittliche NCO-Funktionalität des angewendeten Polyisocyanates entspricht und gleich oder mehr als 2 ist,
***A*** für einen gegebenenfalls substituierten linearen oder verzweigten Linker aus Kohlenstoff, Sauerstoff, Stickstoff, Schwefel, Phosphor und/oder Silizium in der Kette steht
und
***x*** für ein durchschnittlicher molaren Anteil des angebundenen UV-Absorber-Restes steht und weniger als ***m*** ist.

2. UV-Absorber enthaltendes Urethanacrylat gemäß Anspruch 1, **dadurch gekennzeichnet, dass** das Urethanacrylat gemäß Formel (I-1) aufgebaut ist: 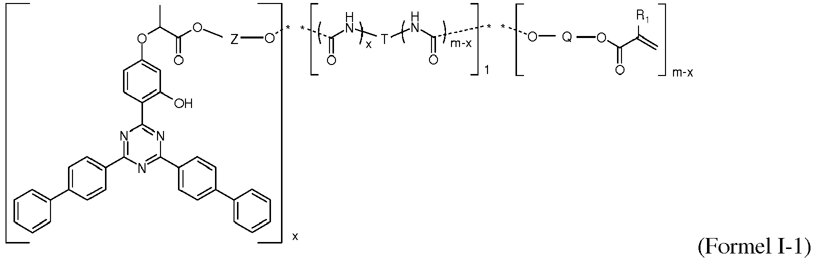 worin
Z für einen gegebenenfalls substituierten linearen oder verzweigten C₁₋₂₀-Alkylen-Rest oder C₁-₂₀-Alkylenether-Rest steht, und
T, Q, m, x und R₁ die vorangehend für die Verbindungen der allgemeinen Formel (I) genannte Bedeutung haben.

3. Verfahren zur Herstellung eines UV-Absorber enthaltenden Urethanacrylats, umfassend die Schritte:
a) Reaktion einer Verbindung der allgemeinen Formel: 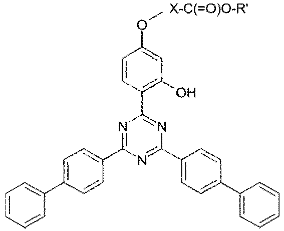 wobei X für verzweigtes oder unverzweigtes C₁₋₂₀-Alkyl steht und R' für verzweigtes oder unverzweigtes C₁₋₂₀Alkyl, C₄₋₁₂-Cycloalkyl oder gegebenenfalls durch C₁₋₁₂-Alkyl, C₁₋₁₂-Alkoxy, CN und/oder Halogen substituiertes C₆₋₁₂-Aryl steht
mit einem mindestens difunktionellen Alkohol;
b) Reaktion des in Schritt a) erhaltenen Produkts mit
bi) einem aliphatischen oder cycloaliphatischen, Isocyanatgruppen enthaltenden Urethanacrylat, welches cyclische Isocyanurat-, Uretdion-, Iminooxadiazindion- oder Oxadiazintrion-Strukturen aufweist oder, im Fall eines cycloaliphatischen Urethanacrylats, weiterhin verzweigte Biuret-Strukturen aufweisen kann,
und/oder mit
bii) einem aliphatischen oder cycloaliphatischen, Isocyanatgruppen enthaltenden Polyisocyanat, welches cyclische Isocyanurat-, Uretdion-, Iminooxadiazindion- oder Oxadiazintrion-Strukturen aufweist oder, im Fall eines cycloaliphatischen Polyisocyanat, weiterhin verzweigte Biuret-Strukturen aufweisen kann,
wobei die Reaktion in Schritt b) weiterhin in Gegenwart eines Hydroxyalkyl(meth)acrylats erfolgt und/oder nach der Reaktion in Schritt b) das erhaltene Produkt weiter mit einem Hydroxyalkyl(meth)acrylat umgesetzt wird.

4. Verfahren gemäß Anspruch 3, **dadurch gekennzeichnet, dass** in Schritt a) in der allgemeinen Formel X für CH(CH₃) steht.

5. Verfahren gemäß Anspruch 3 oder 4, **dadurch gekennzeichnet, dass** in Schritt a) in der allgemeinen Formel R' für n-Octyl oder iso-Octyl steht.

6. Verfahren gemäß wenigstens einem der Anspruch 3 bis 5, **dadurch gekennzeichnet, dass** in Schritt a) der mindestens difunktionelle Alkohol ausgewählt ist aus der Gruppe 2-Butyl-2-ethyl-1,3-propandiol, 2,2-Diethyl-1,3-propandiol, 2-Methyl-1,3-propandiol und/oder 2,2-Dimethyl-1,3-propandiol.

7. Verfahren gemäß wenigstens einem der Ansprüche 3 bis 6, **dadurch gekennzeichnet, dass** das aus Schritt a) erhaltene Produkt ausgewählt ist aus:

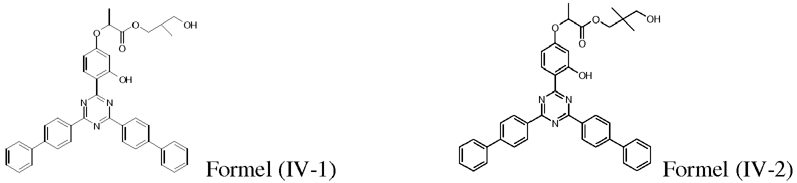

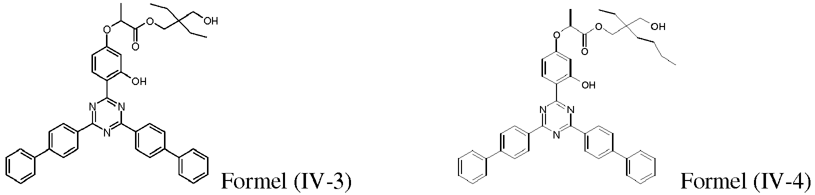

8. Verfahren gemäß wenigstens einem der Ansprüche 3 bis 7, **dadurch gekennzeichnet, dass** in Schritt b) das Isocyanatgruppen enthaltende Urethanacrylat erhältlich ist durch die Reaktion eines 1,6-Hexamethylendiisocyanat-Isocyanurats mit einem Hydroxyalkyl(meth)acrylat.

9. Verfahren gemäß wenigstens einem der Ansprüche 3 bis 8, **dadurch gekennzeichnet, dass** in und/oder nach Schritt b) das Hydroxyalkyl(meth)acrylat ausgewählt ist aus der Gruppe 2-Hydroxyethyl(meth)acrylat, 2-Hydroxypropyl(meth)acrylat, 4-Hydroxybutyl(meth)acrylat und/oder 3-Hydroxy-2,2-dimethylpropyl(meth)acrylat.

10. Beschichtungszusammensetzung, umfassend ein UV-Absorber enthaltendes Urethanacrylat gemäß Anspruch 1 oder 2 und/oder ein durch ein Verfahren gemäß mindestens einem der Ansprüche 3 bis 9 erhältliches, UV-Absorber enthaltendes Urethanacrylat.

11. Beschichtungszusammensetzung gemäß Anspruch 10, umfassend:
i) UV-Absorber enthaltendes Urethanacrylat gemäß Anspruch 1 oder 2 und/oder ein durch ein Verfahren gemäß mindestens einem der Ansprüche 3 bis 9 erhältliches, UV-Absorber enthaltendes Urethanacrylat in 0,1 bis 50 Gew.-Teilen;
ii) 12 bis 70 Gew.-Teile wenigstens eines C₂-C₁₂-Diol-Diacrylats oder C₂-C₁₂-Diol-Dimethacrylats, worin C₂-C1₂ für einen linearen Alkylenrest steht, der gegebenenfalls mit einer Methylgruppe substituiert sein kann oder durch ein oder mehrere Sauerstoff-Atom(e) unterbrochen und optional mit einer oder mehreren Methylgruppe(n) substituiert sein kann,
iii) 12 bis 40 Gew.-Teile wenigstens eines alkoxylierten, bevorzugt ethoxylierten Mono-, Di-, Tri-, Tetra-, Penta- oder Hexaacrylats oder alkoxylierten, bevorzugt ethoxylierten Mono-, Di-, Tri-, Tetra-, Penta- oder Hexamethacrylats,
iv) 0 bis 40 Gew.-Teile wenigstens eines Monomeren ausgewählt aus der Gruppe umfassend Pentaerythrit-Triacrylat, Pentaerythrit-Tetraacrylat, Dipentaerythrit-Tetraacrylat, Dipentaerythrit-Pentaacrylat Dipentaerythrit-Hexaacrylat, Pentaerythrit-Trimethacrylat, Pentaerythrit-Tetramethacrylat, Dipentaerythrit-Tetramethacrylat, Dipentaerythrit-Pentamethacrylat, Dipentaerythrit-Hexamethacrylat und deren möglichen Reaktionsprodukten mit aliphatischen oder aromatischen Diisocyanaten,
v) 5 bis 60 Gew.-Teile wenigstens eines weiteren Mono-, Di- oder Triacrylats oder Mono-, Di-oder Trimethacrylats,
wobei sich die Gewichtsteile der Komponenten i) bis v) zu ≤ 100 Gew.-Teilen addieren, und die Lackzusammensetzung zusätzlich wenigstens noch
vi) 0,1 bis 10 Gew.-Teile wenigstens eines Photoinitiators enthält.

12. Verfahren zum Beschichten eines Substrats, umfassend die Schritte:
- Auftragen einer Beschichtungszusammensetzung gemäß Anspruch 10 oder 11 auf ein Substrat
- Aushärten der zuvor aufgetragenen Beschichtungszusammensetzung durch Bestrahlen mit UV-Licht.

13. Verfahren gemäß Anspruch 12, **dadurch gekennzeichnet, dass** das Substrat ein thermoplastisches Substrat ist.

14. Verfahren gemäß Anspruch 12 oder 13, **dadurch gekennzeichnet, dass** das Substrat ein Polycarbonatsubstrat ist.

15. Beschichtetes Substrat, erhältlich durch ein Verfahren gemäß wenigstens einem der Ansprüche 12 bis 14.

## Claims

1. UV absorber-comprising urethane acrylate of formula (I): 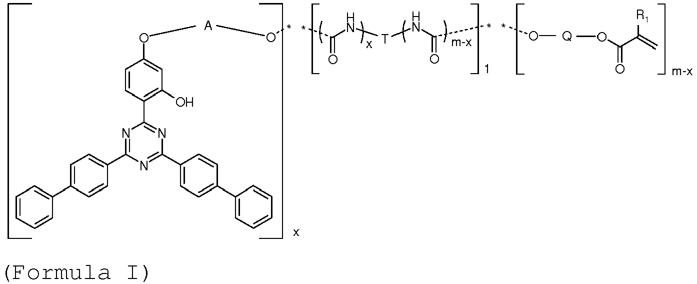 wherein
***R₁*** is a hydrogen or a methyl radical,
***Q*** is a linker of the commercially available hydroxyalkyl (meth)acrylate from the group 2-hydroxyethyl (meth)acrylate, 2-hydroxypropyl (meth)acrylate, 4-hydroxybutyl (meth)acrylate, 3-hydroxy-2,2-dimethylpropyl (meth)acrylate,
***T*** is a nucleus of the commercially available aliphatic and cycloaliphatic polyisocyanates T(NCO)***ₘ*** which have cyclic isocyanurate, uretdione, iminooxadiazinedione or oxadiazinetrione structures, as well as branched biuret structures in the case of cycloaliphatic polyisocyanates,
**m** corresponds to the original average NCO functionality of the polyisocyanate used and is equal to or greater than 2,
**A** represents an optionally substituted linear or branched linker from carbon, oxygen, nitrogen, sulfur, phosphorus and/or silicon in the chain,
and
**x** represents an average molar content of the bonded UV absorber radical and is less than **m.**

2. UV absorber-comprising urethane acrylate according to claim 1, **characterised in that** the urethane acrylate has the structure according to formula (I-1): 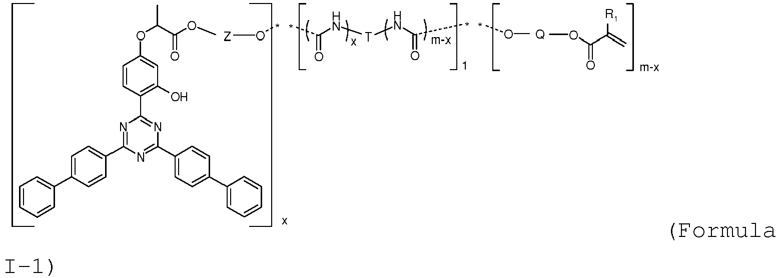 wherein
Z represents an optionally substituted linear or branched C₁₋₂₀-alkylene radical or C₁₋₂₀-alkylene ether radical, and
T, Q, m, x and R₁ have the meanings given above for the compounds of the general formula (I).

3. Process for the preparation of a UV absorber-comprising urethane acrylate, comprising the steps:
a) reacting a compound of the general formula: 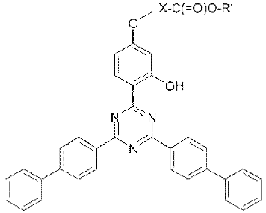 wherein X represents branched or unbranched C₁₋₂₀-alkyl and R' represents branched or unbranched C₁₋₂₀-alkyl, C₄₋₁₂-cycloalkyl, or C₆₋₁₂-aryl optionally substituted by C₁₋₁₂-alkyl, C₁₋₁₂-alkoxy, CN and/or by halogen with an at least difunctional alcohol;
b) reacting the product obtained in step a) with
bi) an aliphatic or cycloaliphatic, isocyanate group-comprising urethane acrylate which has cyclic isocyanurate, uretdione, iminooxadiazinedione or oxadiazinetrione structures or, in the case of a cycloaliphatic urethane acrylate, can further have branched biuret structures,
and/or with
bii) an aliphatic or cycloaliphatic, isocyanate group-comprising polyisocyanate which has cyclic isocyanurate, uretdione, iminooxadiazinedione or oxadiazinetrione structures or, in the case of a cycloaliphatic polyisocyanate, can further have branched biuret structures,
wherein the reaction in step b) further takes place in the presence of a hydroxyalkyl (meth)acrylate and/or after the reaction in step b) the resulting product is further reacted with a hydroxyalkyl (meth)acrylate.

4. Process according to claim 3, **characterised in that** in step a) in the general formula X represents CH(CH₃).

5. Process according to claim 3 or 4, **characterised in that** in step a) in the general formula R' represents n-octyl or isooctyl.

6. Process according to at least one of claims 3 to 5, **characterised in that** in step a) the at least difunctional alcohol is selected from the group 2-butyl-2-ethyl-1,3-propanediol, 2,2-diethyl-1,3-propanediol, 2-methyl-1,3-propanediol and/or 2,2-dimethyl-1,3-propanediol.

7. Process according to at least one of claims 3 to 6, **characterised in that** the product obtained from step a) is selected from: 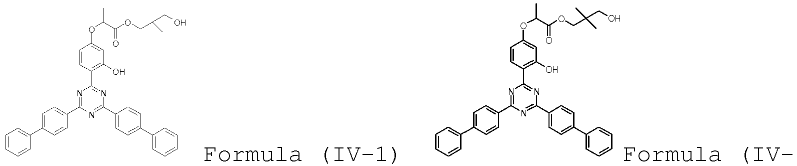 2)

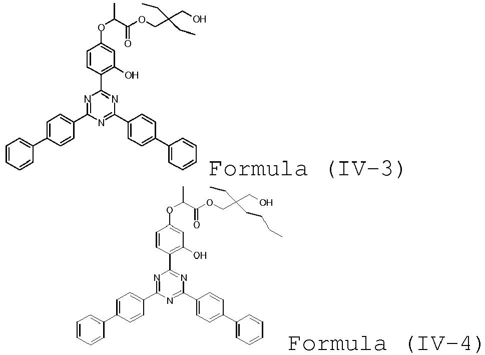

8. Process according to at least one of claims 3 to 7, **characterised in that** in step b) the isocyanate group-comprising urethane acrylate is obtainable by reacting a 1,6-hexamethylene diisocyanate isocyanurate with a hydroxyalkyl (meth)acrylate.

9. Process according to at least one of claims 3 to 8, **characterised in that** in and/or after step b) the hydroxyalkyl (meth)acrylate is selected from the group 2-hydroxyethyl (meth)acrylate, 2-hydroxypropyl (meth)acrylate, 4-hydroxybutyl (meth)acrylate and/or 3-hydroxy-2,2-dimethylpropyl (meth)acrylate.

10. Coating composition comprising a UV absorber-comprising urethane acrylate according to claim 1 or 2 and/or a UV absorber-comprising urethane acrylate obtainable by a process according to at least one of claims 3 to 9.

11. Coating composition according to claim 10, comprising:
i) UV absorber-comprising urethane acrylate according to claim 1 or 2 and/or a UV absorber-comprising urethane acrylate obtainable by a process according to at least one of claims 3 to 9 in an amount of from 0.1 to 50 parts by weight,
ii) from 12 to 70 parts by weight of at least one C₂-C₁₂-diol diacrylate or C₂-C₁₂-diol dimethacrylate, wherein C₂-C₁₂ represents a linear alkylene radical which can optionally be substituted by a methyl group or can be interrupted by one or more oxygen atom(s) and optionally substituted by one or more more methyl group(s),
iii) from 12 to 40 parts by weight of at least one alkoxylated, preferably ethoxylated, mono-, di-, tri-, tetra-, penta- or hexa-acrylate or alkoxylated, preferably ethoxylated, mono-, di-, tri-, tetra-, penta- or hexa-methacrylate,
iv) from 0 to 40 parts by weight of at least one monomer selected from the group comprising pentaerythritol triacrylate, pentaerythritol tetraacrylate, dipentaerythritol tetraacrylate, dipentaerythritol pentaacrylate, dipentaerythritol hexaacrylate, pentaerythritol trimethacrylate, pentaerythritol tetramethacrylate, dipentaerythritol tetramethacrylate, dipentaerythritol pentamethacrylate, dipentaerythritol hexamethacrylate and possible reaction products thereof with aliphatic or aromatic diisocyanates,
v) from 5 to 60 parts by weight of at least one further mono-, di- or tri-acrylate or mono-, di- or tri-methacrylate,
wherein the sum of the parts by weight of components i) to v) is ≤ 100 parts by weight, and the coating composition additionally comprises at least vi) from 0.1 to 10 parts by weight of at least one photoinitiator.

12. Method for coating a substrate, comprising the steps:
- applying a coating composition according to claim 10 or 11 to a substrate,
- curing the previously applied coating composition by irradiation with UV light.

13. Method according to claim 12, **characterised in that** the substrate is a thermoplastic substrate.

14. Method according to claim 12 or 13, **characterised in that** the substrate is a polycarbonate substrate.

15. Coated substrate obtainable by a method according to at least one of claims 12 to 14.

## Revendications

1. Uréthane-acrylate contenant un absorbeur UV, de formule (I) : 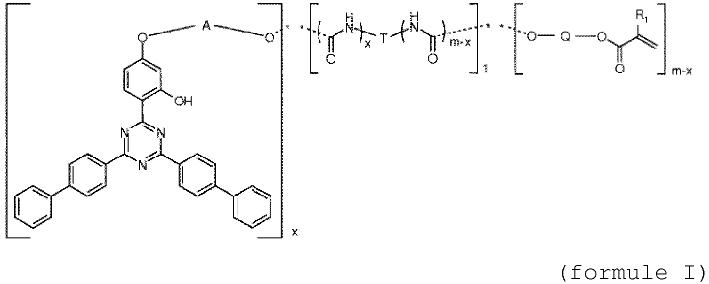 dans laquelle
***R₁*** est un atome d'hydrogène ou un radical méthyle,
***Q*** est un groupe de liaison de type (méth)acrylate d'hydroxyalkyle courant dans le commerce, choisi dans le groupe constitué par le (méth)acrylate de 2-hydroxyéthyle, le (méth)acrylate de 2-hydroxypropyle, le (méth)acrylate de 4-hydroxybutyle, le (méth)acrylate de 3-hydroxy-2,2-diméthylpropyle,
***T*** est un noyau des polyisocyanates aliphatiques et cycloaliphatiques T(NCO)*ₘ* courant dans le commerce, qui comportent des structures cycliques isocyanurate, urétdione, imino-oxadiazinedione ou oxadiazinetrione, ainsi que des structures biuret ramifiées dans le cas des polyisocyanates cycloaliphatiques.
**m** correspond en ce cas à la fonctionnalité NCO moyenne initiale du polyisocyanate utilisé et est égal ou supérieur à 2,
**A** représente un groupe de liaison linéaire ou ramifié, éventuellement substitué, à base de carbone, oxygène, azote, soufre, phosphore et/ou silicium dans la chaîne
et
**x** représente une quantité molaire moyenne du radical absorbeur UV attaché et est inférieur à **m.**

2. Uréthane-acrylate contenant un absorbeur UV selon la revendication 1, **caractérisé en ce que** l'uréthane-acrylate a une structure conforme à la formule (I-1) : 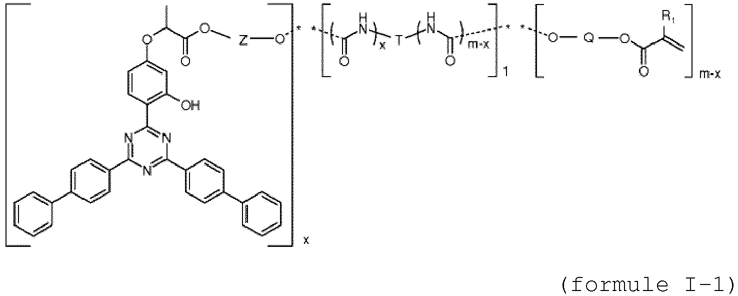 dans laquelle
Z représente un radical alkylène en C₁-C₂₀ ou radical alkylène(C₁-C₂₀)-éther, linéaire ou ramifié, éventuellement substitué,
T, Q, m, x et R₁ ont la signification indiquée précédemment pour les composés de formule générale (I).

3. Procédé pour la préparation d'un uréthane-acrylate contenant un absorbeur UV, comprenant les étapes :
a) réaction d'un composé de formule générale : 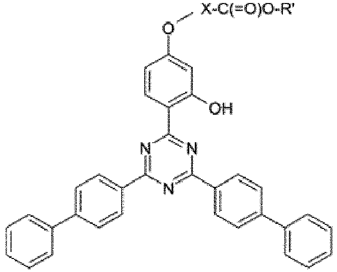 dans laquelle X représente un groupe alkyle en C₁-C₂₀ ramifié ou non ramifié et R' représente un groupe alkyle en C₂-C₂₀ ramifié ou non ramifié, un groupe cycloalkyle en C₄-C₁₂ ou un groupe aryle en C₆-C₁₂ éventuellement substitué par alkyle en C₁-C₁₂, alcoxy en C₁-C₁₂, CN et/ou halogéno
avec un alcool au moins difonctionnel ;
b) réaction du produit obtenu dans l'étape a) avec
b1) un uréthane-acrylate aliphatique ou cycloaliphatique, contenant des groupes isocyanate, qui peut comporter des structures cycliques isocyanurate, urétdione, imino-oxadiazinedione ou oxadiazinetrione ou, dans le cas d'un uréthane-acrylate cycloaliphatique, en outre des structures biuret ramifiées,
et/ou avec
bii) un polyisocyanate aliphatique ou cycloaliphatique, contenant des groupes isocyanate, qui peut comporter des structures cycliques isocyanurate, urétdione, iminooxadiazinedione ou oxadiazinetrione ou, dans le cas d'un polyisocyanate cycloaliphatique, en outre des structures biuret ramifiées,
la réaction dans l'étape b) s'effectuant en outre en présence d'un (méth)acrylate d'hydroxyalkyle et/ou après la réaction dans l'étape b) on fait encore réagir le produit obtenu avec un (méth)acrylate d'hydroxyalkyle.

4. Procédé selon la revendication 3, **caractérisé en ce que** dans l'étape a), dans la formule générale, X représente CH(CH₃).

5. Procédé selon la revendication 3 ou 4, **caractérisé en ce que** dans l'étape a), dans la formule générale, R' représente un groupe n-octyle ou iso-octyle.

6. Procédé selon au moins l'une quelconque des revendications 3 à 5, **caractérisé en ce que** dans l'étape a) l'alcool au moins difonctionnel est choisi dans le groupe constitué par le 2-butyl-2-éthyl-1,3-propanediol, le 2,2-diéthyl-1,3-propanediol, le 2-méthyl-1,3-propanediol et/ou le 2,2-diméthyl-1,3-propanediol.

7. Procédé selon au moins l'une quelconque des revendications 3 à 6, **caractérisé en ce que** le produit obtenu dans l'étape a) est choisi parme :

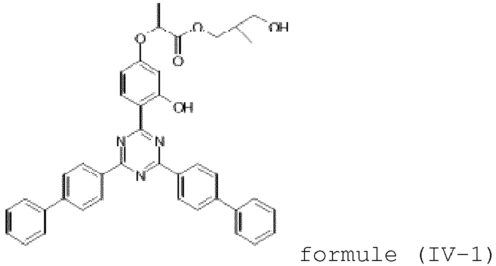

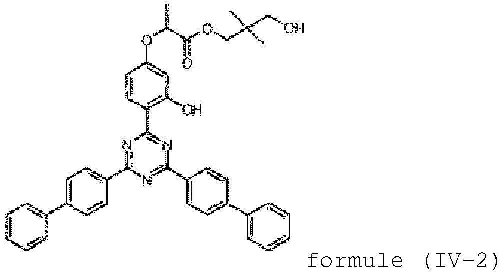

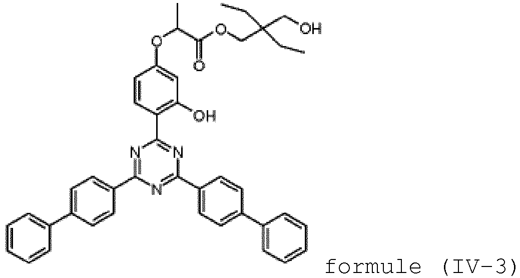

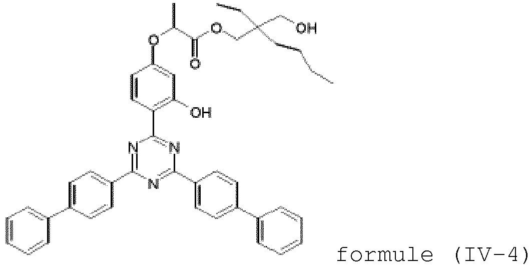

8. Procédé selon au moins l'une quelconque des revendications 3 à 7, **caractérisé en ce que** dans l'étape b), l'uréthane-acrylate contenant des groupes isocyanate peut être obtenu par la réaction d'un 1,6-hexaméthylènediisocyanate-isocyanurate avec un (méth)-acrylate d'hydroxyalkyle.

9. Procédé selon au moins l'une quelconque des revendications 3 à 8, **caractérisé en ce que** dans et/ou après l'étape b), le (méth)acrylate d'hydroxyalkyle est choisi dans le groupe constitué par le (méth)acrylate de 2-hydroxyéthyle, le (méth)acrylate de 2-hydroxypropyle, le (méth)acrylate de 4-hydroxybutyle et/ou le (méth)acrylate de 3-hydroxy-2,2-diméthylpropyle.

10. Composition de revêtement comprenant un uréthane-acrylate contenant un absorbeur UV selon la revendication 1 ou 2 et/ou un uréthane-acrylate contenant un absorbeur UV, pouvant être obtenu par un procédé selon au moins l'une quelconque des revendications 3 à 9.

11. Composition de revêtement selon la revendication 10, comprenant :
i) un uréthane-acrylate contenant un absorbeur UV, selon la revendication 1 ou 2 et/ou un uréthane-acrylate contenant un absorbeur UV, pouvant être obtenu par un procédé selon au moins l'une quelconque des revendications 3 à 9, en 0,1 à 50 parties en poids ;
ii) 12 à 70 parties en poids d'au moins un diol (C₂-C₁₂) -diacrylate en ou diol (C₂-C₁₂)-diméthacrylate, dans lequel C₂-C₁₂ représente un radical alkylène linéaire qui éventuellement peut être substitué par un groupe méthyle ou peut être interrompu par un ou plusieurs atome(s) d'oxygène et en option substitué par un ou plusieurs groupe(s) méthyle,
iii) 12 à 40 parties en poids d'au moins un mono-, di-, tri-, tétra-, penta- ou hexa-acrylate alcoxylé, de préférence éthoxylé ou mono-, di-, tri-, tétra-, penta- ou hexaméthacrylate alcoxylé, de préférence éthoxylé,
iv) 0 à 40 parties en poids d'au moins un monomère choisi dans le groupe comprenant le triacrylate de pentaérythritol, le tétra-acrylate de pentaérythritol, le tétra-acrylate de dipentaérythritol, le penta-acrylate de dipentaérythritol, l'hexa-acrylate de dipentaérythritol, le triméthacrylate de pentaérythritol, le tétraméthacrylate de pentaérythritol, le tétraméthacrylate de dipentaérythritol, le pentaméthacrylate de dipentaérythritol, l'hexaméthacrylate de dipentaérythritol et leurs produits de réaction possibles avec des diisocyanates aliphatiques ou aromatiques,
v) 5 à 60 parties en poids d'au moins un autre mono-, di- ou triacrylate ou mono-, di- ou triméthacrylate,
les parties en poids des composants i) à v) s'ajoutant pour donner ≤ 100 parties en poids, et la composition contenant en outre au moins encore
vi) 0,1 à 10 parties en poids d'au moins un photoamorceur.

12. Procédé pour l'enduction d'un subjectile, comprenant les étapes :
- application d'une composition de revêtement selon la revendication 10 ou 11 sur un subjectile
- durcissement de la composition de revêtement appliquée précédemment, par irradiation avec de la lumière UV.

13. Procédé selon la revendication 12, **caractérisé en ce que** le subjectile est un subjectile thermoplastique.

14. Procédé selon la revendication 12 ou 13, **caractérisé en ce que** le subjectile est un subjectile polycarbonate.

15. Subjectile revêtu, pouvant être obtenu par un procédé selon au moins l'une quelconque des revendications 12 à 14.
